# EUROPEAN PATENT APPLICATION

(11) **EP 2 133 093 A1**
(43) Date of publication of application: **16.12.2009**
(21) Application number: 08721859.0
(22) Date of filing: 12.03.2008
(51) Int. Cl.: A61K 39/395, A61K 45/00, A61P 35/00, A61P 35/02, A61P 43/00

(54) **REMEDY FOR CHEMOTHERAPY-RESISTANT CANCER CONTAINING HLA CLASS I-RECOGNIZING ANTIBODY AS THE ACTIVE INGREDIENT AND USE OF THE SAME**

(30) Priority: 12.03.2007 JP 2007062339
(71) Applicant: Chugai Seiyaku Kabushiki Kaisha, Kita-ku Tokyo 115-8543 (JP)
(72) Inventor: MATSUMOTO, Toshio, Tokushima-shi Tokushima 770-8503 (JP); OZAKI, Shuji, Tokushima-shi Tokushima 770-8503 (JP); ABE, Masahiro, Tokushima-shi Tokushima 770-8503 (JP)
(74) Representative: Vossius & Partner
(86) International application number: PCT/JP2008/054443
(87) International publication number: WO 2008/111597

(57) **Abstract**

The present invention describes therapeutic agents for chemotherapeutic agent-resistant cancers that include an HLA class I-recognizing antibody as an active ingredient. A further objective of the present invention is to provide methods for treating chemotherapeutic agent-resistant cancers that include the step of administering an HLA class I-recognizing antibody to a subject. It is herein demonstrated that cytotoxic activity due to C3B3 diabody is induced at a lower concentration in chemotherapeutic agent-resistant hematological tumor cell lines having high MDR1 and HLA class IA protein expression, than in their parent cell lines. It was also found that when the chemotherapeutic agent-resistant hematological tumor cell line is pretreated with a C3B3 diabody, cell injury associated with sole use of chemotherapeutic agent is enhanced and the amount of pharmaceutical agents taken up into cells are increased. More specifically, it was discovered that the C3B3 diabody exhibits anti-tumor activity on MDR1-expressing tumor cells having high HLA class I expression, and thereby is effective in overcoming drug resistance.

SEQUENCE LISTING

<110> THE UNIVERSITY OF TOKUSHIMA
CHUGAI SEIYAKU KABUSHIKI KAISHA

<120> REMEDY FOR CHEMOTHERAPY-RESISTANT CANCER CONTAINING HLA CLASS
I-RECOGNIZING ANTIBODY AS THE ACTIVE INGREDIENT AND USE OF THE SAME

<130> C1-A0701P

<150> JP 2007-62339
<151> 2007-03-12

<160> 16

<170> PatentIn version 3.4

<210> 1
<211> 375
<212> DNA
<213> Mus musculus

<220>
<221> CDS
<222> (1)..(375)

<400> 1
gaa gtg aag ctg gtg gag tct gag gga ggc tta gtg cag cct gga agt 48
Glu Val Lys Leu Val Glu Ser Glu Gly Gly Leu Val Gln Pro Gly Ser
1               5                   10                  15

tcc atg aaa ctc tcc tgc aca gcc tct gga ttc act ttc agt gac cat 96
Ser Met Lys Leu Ser Cys Thr Ala Ser Gly Phe Thr Phe Ser Asp His
            20                  25                  30

tac atg gct tgg gtc cgc cag gtt cca gaa aag ggt cta gaa tgg gtt 144

Tyr Met Ala Trp Val Arg Gln Val Pro Glu Lys Gly Leu Glu Trp Val
        35                  40                  45

gca gac att aat tat gat ggt agt aga acc tac tat ttg gac tcc ttg 192
Ala Asp Ile Asn Tyr Asp Gly Ser Arg Thr Tyr Tyr Leu Asp Ser Leu
    50                  55                  60

aag agc cgt ttc atc atc tcg aga gac aat gga aag aac att cta tac 240
Lys Ser Arg Phe Ile Ile Ser Arg Asp Asn Gly Lys Asn Ile Leu Tyr
65                  70                  75                  80

cta caa atg agc agt ctg aag tcg gag gac aca gcc acg tat tac tgt 288
Leu Gln Met Ser Ser Leu Lys Ser Glu Asp Thr Ala Thr Tyr Tyr Cys
                85                  90                  95

gca aga gat agg gtt agg tcc tcc tac tat agt aat ctc ttt gct atg 336
Ala Arg Asp Arg Val Arg Ser Ser Tyr Tyr Ser Asn Leu Phe Ala Met
            100                 105                 110

gac tac tgg ggt caa gga att tca gtc acc gtc tcc tca 375
Asp Tyr Trp Gly Gln Gly Ile Ser Val Thr Val Ser Ser
        115                 120                 125

<210> 2
<211> 125
<212> PRT
<213> Mus musculus

<400> 2

Glu Val Lys Leu Val Glu Ser Glu Gly Gly Leu Val Gln Pro Gly Ser
1               5                   10                  15

Ser Met Lys Leu Ser Cys Thr Ala Ser Gly Phe Thr Phe Ser Asp His
            20                  25                  30

Tyr Met Ala Trp Val Arg Gln Val Pro Glu Lys Gly Leu Glu Trp Val
        35                  40                  45

Ala Asp Ile Asn Tyr Asp Gly Ser Arg Thr Tyr Tyr Leu Asp Ser Leu
    50                  55                  60

Lys Ser Arg Phe Ile Ile Ser Arg Asp Asn Gly Lys Asn Ile Leu Tyr
65                  70                  75                  80

Leu Gln Met Ser Ser Leu Lys Ser Glu Asp Thr Ala Thr Tyr Tyr Cys
                85                  90                  95

Ala Arg Asp Arg Val Arg Ser Ser Tyr Tyr Ser Asn Leu Phe Ala Met
            100                 105                 110

Asp Tyr Trp Gly Gln Gly Ile Ser Val Thr Val Ser Ser
        115                 120                 125

<210>  3
<211>  321
<212>  DNA
<213>  Mus musculus

<220>
<221>  CDS
<222>  (1)..(321)

<400>  3

gat atc cag atg aca cag act aca tcc tcc ctt tct gcc tct ctg gga 48
Asp Ile Gln Met Thr Gln Thr Thr Ser Ser Leu Ser Ala Ser Leu Gly
1               5                   10                  15

gac aga gtc acc atc agt tgc agg gca agt cag gat att gcc aat tat 96
Asp Arg Val Thr Ile Ser Cys Arg Ala Ser Gln Asp Ile Ala Asn Tyr
            20                  25                  30

tta aac tgg tat cag cag aaa cca gat gga act gtt aaa ctc ctg atc 144
Leu Asn Trp Tyr Gln Gln Lys Pro Asp Gly Thr Val Lys Leu Leu Ile
        35                  40                  45

tac tac aca tca aga tta cac tca gga gtc cca tca agg ttc agt ggc 192
Tyr Tyr Thr Ser Arg Leu His Ser Gly Val Pro Ser Arg Phe Ser Gly
    50                  55                  60

agt ggg tct ggg aca gat tat tct ctc acc atc agc aac ctg gaa cct 240
Ser Gly Ser Gly Thr Asp Tyr Ser Leu Thr Ile Ser Asn Leu Glu Pro
65                  70                  75                  80

gaa gat att gcc act tac tat tgt cag cag tat agc aag ctt ccg tat 288
Glu Asp Ile Ala Thr Tyr Tyr Cys Gln Gln Tyr Ser Lys Leu Pro Tyr
                85                  90                  95

acg ttc gga tcg ggg acc aag ctg gaa ata aaa 321
Thr Phe Gly Ser Gly Thr Lys Leu Glu Ile Lys
            100                 105

<210> 4
<211> 107
<212> PRT
<213> Mus musculus

<400> 4

Asp Ile Gln Met Thr Gln Thr Thr Ser Ser Leu Ser Ala Ser Leu Gly

1               5                   10                  15

Asp Arg Val Thr Ile Ser Cys Arg Ala Ser Gln Asp Ile Ala Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Lys Leu Pro Tyr
                85                  90                  95

Thr Phe Gly Ser Gly Thr Lys Leu Glu Ile Lys
            100                 105

<210>  5
<211>  711
<212>  DNA
<213>  Artificial

<220>
<223>  An artificially synthesized polynucleotide sequence

<220>

<221>  CDS
<222>  (1)..(711)

<400>  5
gaa gtg aag ctg gtg gag tct gag gga ggc tta gtg cag cct gga agt      48
Glu Val Lys Leu Val Glu Ser Glu Gly Gly Leu Val Gln Pro Gly Ser
1               5                   10                  15

tcc atg aaa ctc tcc tgc aca gcc tct gga ttc act ttc agt gac cat      96
Ser Met Lys Leu Ser Cys Thr Ala Ser Gly Phe Thr Phe Ser Asp His
            20                  25                  30

tac atg gct tgg gtc cgc cag gtt cca gaa aag ggt cta gaa tgg gtt      144
Tyr Met Ala Trp Val Arg Gln Val Pro Glu Lys Gly Leu Glu Trp Val
        35                  40                  45

gca gac att aat tat gat ggt agt aga acc tac tat ttg gac tcc ttg      192
Ala Asp Ile Asn Tyr Asp Gly Ser Arg Thr Tyr Tyr Leu Asp Ser Leu
    50                  55                  60

aag agc cgt ttc atc atc tcg aga gac aat gga aag aac att cta tac      240
Lys Ser Arg Phe Ile Ile Ser Arg Asp Asn Gly Lys Asn Ile Leu Tyr
65                  70                  75                  80

cta caa atg agc agt ctg aag tcg gag gac aca gcc acg tat tac tgt      288
Leu Gln Met Ser Ser Leu Lys Ser Glu Asp Thr Ala Thr Tyr Tyr Cys
                85                  90                  95

gca aga gat agg gtt agg tcc tcc tac tat agt aat ctc ttt gct atg      336
Ala Arg Asp Arg Val Arg Ser Ser Tyr Tyr Ser Asn Leu Phe Ala Met
            100                 105                 110

gac tac tgg ggt caa gga att tca gtc acc gtc tcc tca ggt gga ggc      384
Asp Tyr Trp Gly Gln Gly Ile Ser Val Thr Val Ser Ser Gly Gly Gly
        115                 120                 125

ggt agc gat atc cag atg aca cag act aca tcc tcc ctt tct gcc tct      432

Gly Ser Asp Ile Gln Met Thr Gln Thr Thr Ser Ser Leu Ser Ala Ser
    130                 135                 140

ctg gga gac aga gtc acc atc agt tgc agg gca agt cag gat att gcc      480
Leu Gly Asp Arg Val Thr Ile Ser Cys Arg Ala Ser Gln Asp Ile Ala
145                 150                 155                 160

aat tat tta aac tgg tat cag cag aaa cca gat gga act gtt aaa ctc      528
Asn Tyr Leu Asn Trp Tyr Gln Gln Lys Pro Asp Gly Thr Val Lys Leu
                165                 170                 175

ctg atc tac tac aca tca aga tta cac tca gga gtc cca tca agg ttc      576
Leu Ile Tyr Tyr Thr Ser Arg Leu His Ser Gly Val Pro Ser Arg Phe
            180                 185                 190

agt ggc agt ggg tct ggg aca gat tat tct ctc acc atc agc aac ctg      624
Ser Gly Ser Gly Ser Gly Thr Asp Tyr Ser Leu Thr Ile Ser Asn Leu
        195                 200                 205

gaa cct gaa gat att gcc act tac tat tgt cag cag tat agc aag ctt      672
Glu Pro Glu Asp Ile Ala Thr Tyr Tyr Cys Gln Gln Tyr Ser Lys Leu
    210                 215                 220

ccg tat acg ttc gga tcg ggg acc aag ctg gaa ata aaa                  711
Pro Tyr Thr Phe Gly Ser Gly Thr Lys Leu Glu Ile Lys
225                 230                 235

<210>  6
<211>  237
<212>  PRT
<213>  Artificial

<220>
<223>  Synthetic Construct

<400>  6

Glu Val Lys Leu Val Glu Ser Glu Gly Gly Leu Val Gln Pro Gly Ser
1               5                   10                  15

Ser Met Lys Leu Ser Cys Thr Ala Ser Gly Phe Thr Phe Ser Asp His
            20                  25                  30

Tyr Met Ala Trp Val Arg Gln Val Pro Glu Lys Gly Leu Glu Trp Val
        35                  40                  45

Ala Asp Ile Asn Tyr Asp Gly Ser Arg Thr Tyr Tyr Leu Asp Ser Leu
    50                  55                  60

Lys Ser Arg Phe Ile Ile Ser Arg Asp Asn Gly Lys Asn Ile Leu Tyr
65                  70                  75                  80

Leu Gln Met Ser Ser Leu Lys Ser Glu Asp Thr Ala Thr Tyr Tyr Cys
                85                  90                  95

Ala Arg Asp Arg Val Arg Ser Ser Tyr Tyr Ser Asn Leu Phe Ala Met
            100                 105                 110

Asp Tyr Trp Gly Gln Gly Ile Ser Val Thr Val Ser Ser Gly Gly Gly
        115                 120                 125

Gly Ser Asp Ile Gln Met Thr Gln Thr Thr Ser Ser Leu Ser Ala Ser
    130                 135                 140

Leu Gly Asp Arg Val Thr Ile Ser Cys Arg Ala Ser Gln Asp Ile Ala
145                 150                 155                 160

Asn Tyr Leu Asn Trp Tyr Gln Gln Lys Pro Asp Gly Thr Val Lys Leu
                165                 170                 175

Leu Ile Tyr Tyr Thr Ser Arg Leu His Ser Gly Val Pro Ser Arg Phe
            180                 185                 190

Ser Gly Ser Gly Ser Gly Thr Asp Tyr Ser Leu Thr Ile Ser Asn Leu
        195                 200                 205

Glu Pro Glu Asp Ile Ala Thr Tyr Tyr Cys Gln Gln Tyr Ser Lys Leu
    210                 215                 220

Pro Tyr Thr Phe Gly Ser Gly Thr Lys Leu Glu Ile Lys
225                 230                 235

<210>  7
<211>  5
<212>  PRT
<213>  Mus musculus

<400>  7

Asp His Tyr Met Ala
1               5

<210>  8
<211>  17

<212>  PRT
<213>  Mus musculus

<400>  8

Asp Ile Asn Tyr Asp Gly Ser Arg Thr Tyr Tyr Leu Asp Ser Leu Lys
1               5                   10                  15

Ser

<210>  9
<211>  16
<212>  PRT
<213>  Mus musculus

<400>  9

Asp Arg Val Arg Ser Ser Tyr Tyr Ser Asn Leu Phe Ala Met Asp Tyr
1               5                   10                  15

<210>  10
<211>  11
<212>  PRT
<213>  Mus musculus

<400>  10

Arg Ala Ser Gln Asp Ile Ala Asn Tyr Leu Asn
1               5                   10

<210>  11
<211>  7

<212>  PRT
<213>  Mus musculus

<400>  11

Tyr Thr Ser Arg Leu His Ser
1               5

<210>  12
<211>  9
<212>  PRT
<213>  Mus musculus

<400>  12

Gln Gln Tyr Ser Lys Leu Pro Tyr Thr
1               5

<210>  13
<211>  20
<212>  DNA
<213>  Artificial

<220>
<223>  An artificially synthesized primer sequence

<400>  13
cccatcattg caatagcagg                                                    20

<210>  14
<211>  20
<212>  DNA
<213>  Artificial

<220>
<223>  An artificially synthesized primer sequence

<400>  14
agtcctcgtc ttcaaacttg                                                    20


<210>  15
<211>  20
<212>  DNA
<213>  Artificial

<220>
<223>  An artificially synthesized primer sequence

<400>  15
acccccactg aaaaagatga                                                    20


<210>  16
<211>  20
<212>  DNA
<213>  Artificial

<220>
<223>  An artificially synthesized primer sequence

<400>  16
gtagtacctc caaacttcta                                                    20

SEQUENCE LISTING

<110> THE UNIVERSITY OF TOKUSHIMA
CHUGAI SEIYAKU KABUSHIKI KAISHA

<120> Therapeutic agent for chemical therapy resistant cancer
comprising an antibody that recognize HLA class I as an active
ingredient

<130> C1-A0701P

<150> JP 2007-62339
<151> 2007-03-12

<160> 16

<170> PatentIn version 3.4

<210> 1
<211> 375
<212> DNA
<213> Mus musculus

<220>
<221> CDS
<222> (1)..(375)

<400> 1
gaa gtg aag ctg gtg gag tct gag gga ggc tta gtg cag cct gga agt 48
Glu Val Lys Leu Val Glu Ser Glu Gly Gly Leu Val Gln Pro Gly Ser
1               5                   10                  15

tcc atg aaa ctc tcc tgc aca gcc tct gga ttc act ttc agt gac cat 96
Ser Met Lys Leu Ser Cys Thr Ala Ser Gly Phe Thr Phe Ser Asp His
            20                  25                  30

tac atg gct tgg gtc cgc cag gtt cca gaa aag ggt cta gaa tgg gtt 144
Tyr Met Ala Trp Val Arg Gln Val Pro Glu Lys Gly Leu Glu Trp Val
        35                  40                  45

gca gac att aat tat gat ggt agt aga acc tac tat ttg gac tcc ttg 192
Ala Asp Ile Asn Tyr Asp Gly Ser Arg Thr Tyr Tyr Leu Asp Ser Leu
    50                  55                  60

aag agc cgt ttc atc atc tcg aga gac aat gga aag aac att cta tac 240
Lys Ser Arg Phe Ile Ile Ser Arg Asp Asn Gly Lys Asn Ile Leu Tyr
65                  70                  75                  80

cta caa atg agc agt ctg aag tcg gag gac aca gcc acg tat tac tgt 288
Leu Gln Met Ser Ser Leu Lys Ser Glu Asp Thr Ala Thr Tyr Tyr Cys
                85                  90                  95

gca aga gat agg gtt agg tcc tcc tac tat agt aat ctc ttt gct atg 336
Ala Arg Asp Arg Val Arg Ser Ser Tyr Tyr Ser Asn Leu Phe Ala Met
            100                 105                 110

gac tac tgg ggt caa gga att tca gtc acc gtc tcc tca 375
Asp Tyr Trp Gly Gln Gly Ile Ser Val Thr Val Ser Ser
        115                 120                 125

<210> 2
<211> 125
<212> PRT
<213> Mus musculus

<400> 2

Glu Val Lys Leu Val Glu Ser Glu Gly Gly Leu Val Gln Pro Gly Ser
1               5                   10                  15

Ser Met Lys Leu Ser Cys Thr Ala Ser Gly Phe Thr Phe Ser Asp His
            20                  25                  30

Tyr Met Ala Trp Val Arg Gln Val Pro Glu Lys Gly Leu Glu Trp Val
        35                  40                  45

Ala Asp Ile Asn Tyr Asp Gly Ser Arg Thr Tyr Tyr Leu Asp Ser Leu
    50                  55                  60

Lys Ser Arg Phe Ile Ile Ser Arg Asp Asn Gly Lys Asn Ile Leu Tyr
65                  70                  75                  80

Leu Gln Met Ser Ser Leu Lys Ser Glu Asp Thr Ala Thr Tyr Tyr Cys
                85                  90                  95

Ala Arg Asp Arg Val Arg Ser Ser Tyr Tyr Ser Asn Leu Phe Ala Met
            100                 105                 110

Asp Tyr Trp Gly Gln Gly Ile Ser Val Thr Val Ser Ser
        115                 120                 125

<210>  3
<211>  321
<212>  DNA
<213>  Mus musculus

<220>
<221>  CDS
<222>  (1)..(321)

<400>  3
gat atc cag atg aca cag act aca tcc tcc ctt tct gcc tct ctg gga      48
Asp Ile Gln Met Thr Gln Thr Thr Ser Ser Leu Ser Ala Ser Leu Gly
1               5                   10                  15

gac aga gtc acc atc agt tgc agg gca agt cag gat att gcc aat tat      96
Asp Arg Val Thr Ile Ser Cys Arg Ala Ser Gln Asp Ile Ala Asn Tyr
            20                  25                  30

tta aac tgg tat cag cag aaa cca gat gga act gtt aaa ctc ctg atc      144
Leu Asn Trp Tyr Gln Gln Lys Pro Asp Gly Thr Val Lys Leu Leu Ile
        35                  40                  45

tac tac aca tca aga tta cac tca gga gtc cca tca agg ttc agt ggc      192
Tyr Tyr Thr Ser Arg Leu His Ser Gly Val Pro Ser Arg Phe Ser Gly
    50                  55                  60

agt ggg tct ggg aca gat tat tct ctc acc atc agc aac ctg gaa cct      240
Ser Gly Ser Gly Thr Asp Tyr Ser Leu Thr Ile Ser Asn Leu Glu Pro
65                  70                  75                  80

gaa gat att gcc act tac tat tgt cag cag tat agc aag ctt ccg tat      288
Glu Asp Ile Ala Thr Tyr Tyr Cys Gln Gln Tyr Ser Lys Leu Pro Tyr
                85                  90                  95

acg ttc gga tcg ggg acc aag ctg gaa ata aaa                          321
Thr Phe Gly Ser Gly Thr Lys Leu Glu Ile Lys

            100                 105

<210> 4
<211> 107
<212> PRT
<213> Mus musculus

<400> 4

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ala Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Lys Leu Pro Tyr
                85                  90                  95

Thr Phe Gly Ser Gly Thr Lys Leu Glu Ile Lys
            100                 105

<210> 5
<211> 711
<212> DNA
<213> Artificial

<220>
<223> An artificially synthesized polynucleotide sequence

<220>
<221> CDS
<222> (1)..(711)

<400> 5
gaa gtg aag ctg gtg gag tct gag gga ggc tta gtg cag cct gga agt 48
Glu Val Lys Leu Val Glu Ser Glu Gly Gly Leu Val Gln Pro Gly Ser
1               5                   10                  15

tcc atg aaa ctc tcc tgc aca gcc tct gga ttc act ttc agt gac cat 96
Ser Met Lys Leu Ser Cys Thr Ala Ser Gly Phe Thr Phe Ser Asp His
            20                  25                  30

tac atg gct tgg gtc cgc cag gtt cca gaa aag ggt cta gaa tgg gtt 144
Tyr Met Ala Trp Val Arg Gln Val Pro Glu Lys Gly Leu Glu Trp Val
        35                  40                  45

gca gac att aat tat gat ggt agt aga acc tac tat ttg gac tcc ttg 192
Ala Asp Ile Asn Tyr Asp Gly Ser Arg Thr Tyr Tyr Leu Asp Ser Leu
    50                  55                  60

aag agc cgt ttc atc atc tcg aga gac aat gga aag aac att cta tac 240

Lys Ser Arg Phe Ile Ile Ser Arg Asp Asn Gly Lys Asn Ile Leu Tyr
65                  70                  75                  80

cta caa atg agc agt ctg aag tcg gag gac aca gcc acg tat tac tgt 288
Leu Gln Met Ser Ser Leu Lys Ser Glu Asp Thr Ala Thr Tyr Tyr Cys
                85                  90                  95

gca aga gat agg gtt agg tcc tcc tac tat agt aat ctc ttt gct atg 336
Ala Arg Asp Arg Val Arg Ser Ser Tyr Tyr Ser Asn Leu Phe Ala Met
            100                 105                 110

gac tac tgg ggt caa gga att tca gtc acc gtc tcc tca ggt gga ggc 384
Asp Tyr Trp Gly Gln Gly Ile Ser Val Thr Val Ser Ser Gly Gly Gly
        115                 120                 125

ggt agc gat atc cag atg aca cag act aca tcc tcc ctt tct gcc tct 432
Gly Ser Asp Ile Gln Met Thr Gln Thr Thr Ser Ser Leu Ser Ala Ser
    130                 135                 140

ctg gga gac aga gtc acc atc agt tgc agg gca agt cag gat att gcc 480
Leu Gly Asp Arg Val Thr Ile Ser Cys Arg Ala Ser Gln Asp Ile Ala
145                 150                 155                 160

aat tat tta aac tgg tat cag cag aaa cca gat gga act gtt aaa ctc 528
Asn Tyr Leu Asn Trp Tyr Gln Gln Lys Pro Asp Gly Thr Val Lys Leu
                165                 170                 175

ctg atc tac tac aca tca aga tta cac tca gga gtc cca tca agg ttc 576
Leu Ile Tyr Tyr Thr Ser Arg Leu His Ser Gly Val Pro Ser Arg Phe
            180                 185                 190

agt ggc agt ggg tct ggg aca gat tat tct ctc acc atc agc aac ctg 624
Ser Gly Ser Gly Ser Gly Thr Asp Tyr Ser Leu Thr Ile Ser Asn Leu
        195                 200                 205

gaa cct gaa gat att gcc act tac tat tgt cag cag tat agc aag ctt 672
Glu Pro Glu Asp Ile Ala Thr Tyr Tyr Cys Gln Gln Tyr Ser Lys Leu
    210                 215                 220

ccg tat acg ttc gga tcg ggg acc aag ctg gaa ata aaa 711
Pro Tyr Thr Phe Gly Ser Gly Thr Lys Leu Glu Ile Lys
225                 230                 235

<210> 6
<211> 237
<212> PRT
<213> Artificial

<220>
<223> Synthetic Construct

<400> 6

Glu Val Lys Leu Val Glu Ser Glu Gly Gly Leu Val Gln Pro Gly Ser
1               5                   10                  15

Ser Met Lys Leu Ser Cys Thr Ala Ser Gly Phe Thr Phe Ser Asp His
            20                  25                  30

Tyr Met Ala Trp Val Arg Gln Val Pro Glu Lys Gly Leu Glu Trp Val
        35                  40                  45

Ala Asp Ile Asn Tyr Asp Gly Ser Arg Thr Tyr Tyr Leu Asp Ser Leu
    50                  55                  60

Lys Ser Arg Phe Ile Ile Ser Arg Asp Asn Gly Lys Asn Ile Leu Tyr
65                  70                  75                  80

Leu Gln Met Ser Ser Leu Lys Ser Glu Asp Thr Ala Thr Tyr Tyr Cys
                85                  90                  95

Ala Arg Asp Arg Val Arg Ser Ser Tyr Tyr Ser Asn Leu Phe Ala Met
            100                 105                 110

Asp Tyr Trp Gly Gln Gly Ile Ser Val Thr Val Ser Ser Gly Gly Gly
        115                 120                 125

Gly Ser Asp Ile Gln Met Thr Gln Thr Thr Ser Ser Leu Ser Ala Ser
    130                 135                 140

Leu Gly Asp Arg Val Thr Ile Ser Cys Arg Ala Ser Gln Asp Ile Ala
145                 150                 155                 160

Asn Tyr Leu Asn Trp Tyr Gln Gln Lys Pro Asp Gly Thr Val Lys Leu
                165                 170                 175

Leu Ile Tyr Tyr Thr Ser Arg Leu His Ser Gly Val Pro Ser Arg Phe
            180                 185                 190

Ser Gly Ser Gly Ser Gly Thr Asp Tyr Ser Leu Thr Ile Ser Asn Leu
        195                 200                 205

Glu Pro Glu Asp Ile Ala Thr Tyr Tyr Cys Gln Gln Tyr Ser Lys Leu
    210                 215                 220

Pro Tyr Thr Phe Gly Ser Gly Thr Lys Leu Glu Ile Lys
225                 230                 235

<210>  7
<211>  5
<212>  PRT
<213>  Mus musculus

<400>  7

Asp His Tyr Met Ala
1               5

<210>  8
<211>  17
<212>  PRT
<213>  Mus musculus

<400>  8

Asp Ile Asn Tyr Asp Gly Ser Arg Thr Tyr Tyr Leu Asp Ser Leu Lys
1               5                   10                  15

Ser

<210> 9
<211> 16
<212> PRT
<213> Mus musculus

<400> 9

Asp Arg Val Arg Ser Ser Tyr Tyr Ser Asn Leu Phe Ala Met Asp Tyr
1               5                   10                  15


<210> 10
<211> 11
<212> PRT
<213> Mus musculus

<400> 10

Arg Ala Ser Gln Asp Ile Ala Asn Tyr Leu Asn
1               5                   10


<210> 11
<211> 7
<212> PRT
<213> Mus musculus

<400> 11

Tyr Thr Ser Arg Leu His Ser
1               5


<210> 12
<211> 9
<212> PRT
<213> Mus musculus

<400> 12

Gln Gln Tyr Ser Lys Leu Pro Tyr Thr
1               5


<210> 13
<211> 20
<212> DNA
<213> Artificial

<220>
<223> An artificially synthesized primer sequence

<400> 13
cccatcattg caatagcagg 20


<210> 14
<211> 20
<212> DNA
<213> Artificial

<220>
<223> An artificially synthesized primer sequence

<400> 14
agtcctcgtc ttcaaacttg 20

<210> 15
<211> 20
<212> DNA
<213> Artificial

<220>
<223> An artificially synthesized primer sequence

<400> 15
acccccactg aaaaagatga 20


<210> 16
<211> 20
<212> DNA
<213> Artificial

<220>
<223> An artificially synthesized primer sequence

<400> 16
gtagtacctc caaacttcta 20

## Description

### Technical Field

The present invention relates to therapeutic agents for chemotherapeutic agent-resistant cancer that include, as an active ingredient, an HLA class I-recognizing antibody and uses of such agents. The present invention also relates to methods for treating chemotherapeutic agent-resistant cancer that include the step of administering an HLA class I-recognizing antibody to a subject.

### Background Art

HLA, an important immune response molecule, is involved in recognizing and eliminating exogenous antigens, bacteria, virus-infected cells, and other of such foreign substances. The main role of the HLA molecule is to present antigenic peptides, which are made up of about eight to ten amino acid residues, produced inside cells to CD8⁺T cells. Accordingly, the HLA molecule plays a very important role in the immune response, and in immune tolerance induced by the peptide presentation. HLA molecules are categorized into class I and class II. Class I molecules form a heterodimer of a12-KD β2 microglobulin (β2M) and a 45-KD α-chain composed of three domains, α1-3. Class II molecules form a heterodimer of a 30-34 KD α-chain composed of two domains, α1 and α2, and a 26-29 KD β-chain composed of two domains, β1 and β2. HLA class I (HLA-I) molecules are known to be further classified into HLA-A, B, C, and such (hereinafter, HLA-A is also called as "HLA class I A (HLA-IA)").

To date, cell growth-suppressing and cell death-inducing effects have been reported for lymphocytes that are ligated with an anti-HLA class IA antibody, suggesting that HLA molecules may be signal transduction molecules. For example, it has been reported that cell growth of activated lymphocytes is suppressed by the B9.12.1 antibody against the α1 domain of human HLA class IA, W6/32 antibody against the α2 domain, and TP25.99 and A1.4 antibodies against the α3 domain (Non-patent Documents 1 and 2). In addition, two antibodies against the α1 domain, MoAb90 and YTH862, have been reported to induce apoptosis in activated lymphocytes (Non-patent Documents 2, 3, and 4). Apoptosis induced by these two antibodies has been shown to be a caspase-mediated reaction (Non-patent Document 4). Accordingly, there is speculation that HLA class IA expressed in lymphocytes is involved in apoptosis signal transduction.

Furthermore, 5H7 antibody against the α3 domain of human HLA class IA (Non-patent Document 5), and the RE2 antibody against the α2 domain of mouse MHC class I (Non-patent Document 6) have been also reported to induce cell death in activated lymphocytes and the like.

The monoclonal antibody 2D7 (Non-patent Document 8), obtained by immunizing human myeloma cells, is also reported to be an HLA class IA-recognizing antibody. More particularly, when made into a low-molecular-weight antibody (diabody), it can quickly induce severe cell death in human myeloma cells. Furthermore, the monoclonal antibody C3B3, obtained by immunizing mice with cells coexpressing human HLA class IA and human β2M molecules (Patent Document 5), is an antibody that recognizes HLA class I antigen α2 domain; it also shows strong cytotoxic activity when cross-linked with an anti-mouse IgG antibody. Furthermore, when modified into a low-molecular-weight antibody (C3B3 diabody), the C3B3 diabody (C3B3-DB), when used alone showed stronger antitumor effects than 2D7 diabody used alone (Patent Document 5).

Because they show strong cell death-inducing activity in various human myeloma cell lines and activated lymphocytes, and demonstrate significant survival benefit in multiple myeloma model mice generated by transplanting human myeloma cell line to mice, the 2D7 diabody and the C3B3 diabody are presently under development as therapeutic agents for myeloma (Patent Documents 1 to 5, and Non-patent Document 7). Further advances in treatments utilizing cell death induction involving HLA class I are expected to lead to the development of highly effective pharmaceuticals for myeloma and the like.

Chemotherapy for malignant tumors is known to induce the expression of MDR1 (P glycoprotein, Multidrug resistance protein 1, ATP-binding cassette sub-family B member 1 (ABCB1)), a protein belonging to the ATP-binding cassette (ABC) transporter superfamily in tumor cells, which is problematic. This MDR1 expression is involved in the acquisition of drug (chemotherapeutic agent) resistance by tumor cells, and, as such, is a factor in diminishing the effects of subsequent chemotherapy. Therefore, developing therapeutic strategies that can overcome drug resistance in MDR1-expressing tumors is an important objective. Since MDR1 acts to excrete pharmaceutical agents from the cell interior to the cell exterior, attempts to use therapeutic methods to suppress such action, for example using verapamil, an ABC transporter inhibitor, have been made; however, its clinical effectiveness has yet to be established. On the other hand, enhanced expression of HLA class I molecules in MDR1-expressing tumors has been reported in the literature (Non-patent Documents 9 and 10).

Prior art literature related to the present invention are listed below.
Patent Document 1: WO2004/033499
Patent Document 2: WO2005/056603
Patent Document 3: WO2005/100560
Patent Document 4: WO2006/123724
Patent Document 5: PCT/JP2007/063946
Non-patent Document 1: Fayen et al., Int. Immunol. (1998) 10: 1347-1358
Non-patent Document 2: Genestier et al., Blood (1997) 90: 3629-3639
Non-patent Document 3: Genestier et al., Blood (1997) 90: 726-735
Non-patent Document 4: Genestier et al., J. Biol. Chem. (1998) 273: 5060-5066
Non-patent Document 5: Woodle et al., J. Immunol. (1997) 158: 2156-2164
Non-patent Document 6: Matsuoka et al., J. Exp. Med. (1995) 181: 2007-2015
Non-patent Document 7: Kimura, et al., Biochem. Biophys. Res. Commun. (2004) 325: 1201-1209
Non-patent Document 8: Oka, T., "Sankyo Seimei-kagaku-zaidan Kenkyu Hokoku-shu (Research Reports of the Sankyo Life Science Foundation)" (1998) 12: 46-56
Non-patent Document 9: Neoplasma (2003) 50 (2): 91-96
Non-patent Document 10: Prados J. et al., Neoplasm (2006) 53 (3): 226-231
Non-patent Document 11: Journal of Internal Medicine (2000) 247: 521-534

### Disclosure of the Invention

### [Problems to be Solved by the Invention]

The present invention was achieved in view of the above circumstances. An objective of the present invention is to provide a therapeutic agent for chemotherapeutic agent-resistant cancer, such agents including an HLA class I-recognizing antibody as an active ingredient. A further objective of the present invention is to provide methods for treating chemotherapeutic agent-resistant cancer, such methods including the step of administering an HLA class I-recognizing antibody to a subject.

### [Means for Solving the Problems]

To address the above-mentioned objective, the effects of a C3B3 diabody (C3B3-DB), a low-molecular-weight antibody of the C3B3 antibody, on drug-resistant tumor cells were examined herein.

First, it was herein confirmed that MDR1 and HLA-1A (HLA class IA) proteins are highly expressed in a chemotherapeutic agent (vineristine)-resistant hematological tumor cell line, and that their expression is higher in hematological tumor patient deriving tumor cells obtained at the first medical examination than those obtained at the time of recurrence.

Next, it was discovered that the cytotoxic activity of the C3B3 diabody is induced at a lower concentration in this chemotherapeutic agent-resistant hematological tumor cell line than in the parent cell line. It was also discovered that when the chemotherapeutic agent-resistant hematological tumor cell line is pretreated with a C3B3 diabody (0.1 µg/mL) for six hours, cell injury led by the chemotherapeutic agent (vincristine) is enhanced as compared to the case in which pretreatment was not performed. Furthermore, chemotherapeutic agent-resistant hematological tumor cell line pretreated with a C3B3 diabody (0.1 µg/mL) was found to have a decreased expression of MDR1 protein on the cell surface in some cells and to cause an increase in the amount of pharmaceutical agent taken into the cells.

More specifically, the C3B3 diabody was found to exert antitumor activity against MDR1-expressing tumor cells that highly express HLA class I, and thereby act to overcome drug resistance. These findings led to the completion of the present invention.

More specifically, the present invention provides the following [1] to [43]:
[1] a therapeutic agent for chemotherapeutic agent-resistant cancer, comprising an HLA class I-recognizing antibody as an active ingredient;
[2] the therapeutic agent for chemotherapeutic agent-resistant cancer of [1], wherein the antibody is a low-molecular-weight antibody;
[3] the therapeutic agent for chemotherapeutic agent-resistant cancer of [1] or [2], in combination with a chemotherapeutic agent;
[4] the therapeutic agent for chemotherapeutic agent-resistant cancer of any one of [1] to [3], wherein the chemotherapeutic agent-resistant cancer is a hematological tumor;
[5] a potentiator of a chemotherapeutic agent comprising an HLA class I-recognizing antibody as an active ingredient;
[6] the potentiator of a chemotherapeutic agent of [5], wherein the antibody is a low-molecular-weight antibody;
[7] the potentiator of a chemotherapeutic agent of [5] or [6], in combination with a chemotherapeutic agent;
[8] the potentiator of a chemotherapeutic agent of any one of [5] to [7], wherein the cancer treated by a chemotherapeutic agent is a chemotherapeutic agent-resistant cancer;
[9] the potentiator of a chemotherapeutic agent of [8], wherein the chemotherapeutic agent-resistant cancer is a hematological tumor;
[10] a pharmaceutical composition comprising an HLA class I-recognizing antibody as an active ingredient, in combination with a chemotherapeutic agent;
[11] a pharmaceutical composition for cancer therapy comprising an HLA class I-recognizing antibody as an active ingredient, in combination with a chemotherapeutic agent;
[12] the pharmaceutical composition of [10] or [11], wherein the antibody is a low-molecular-weight antibody;
[13] a pharmaceutical composition for cancer therapy comprising a chemotherapeutic agent and an HLA class I-recognizing antibody as active ingredients;
[14] the pharmaceutical composition for cancer therapy of [13], wherein the antibody is a low-molecular-weight antibody;
[15] the pharmaceutical composition of any one of [10] to [14], wherein the cancer to be treated with a chemotherapeutic agent is a chemotherapeutic agent-resistant cancer;
[16] the pharmaceutical composition of [15], wherein the chemotherapeutic agent-resistant cancer is a hematological tumor;
[17] a method for treating chemotherapeutic agent-resistant cancer, wherein the method comprises the step of administering an HLA class I-recognizing antibody to a subject;
[18] the method of [17], wherein the antibody is a low-molecular-weight antibody;
[19] the method of [17] or [18], wherein a chemotherapeutic agent is used in combination;
[20] the method of any one of [17] to [19], wherein the chemotherapeutic agent-resistant cancer is a hematological tumor;
[21] a method for strengthening the effect of a chemotherapeutic agent, wherein the method comprises the step of administering an HLA class I-recognizing antibody to a subject;
[22] the method of [21], wherein the antibody is a low-molecular-weight antibody;
[23] the method of [21] or [22], wherein the chemotherapeutic agent is used in combination;
[24] the method of any one of [21] to [23], wherein a cancer to be treated with a chemotherapeutic agent is a chemotherapeutic agent-resistant cancer;
[25] the method [24], wherein the chemotherapeutic agent-resistant cancer is a hematological tumor;
[26] a method for treating cancer, said method comprising the step of administering to a subject in need thereof a chemotherapeutic agent in combination with an HLA class I-recognizing antibody;
[27] the method of [26], wherein the antibody is a low-molecular-weight antibody;
[28] the method of [26] or [27], wherein the cancer is a chemotherapeutic agent-resistant cancer;
[29] the method of [28], wherein the chemotherapeutic agent-resistant cancer is a hematological tumor;
[30] use of an HLA class I-recognizing antibody in the manufacture of a therapeutic agent for chemotherapeutic agent-resistant cancer;
[31] use of [30], wherein the antibody is a low-molecular-weight antibody;
[32] use of [30] or [31], wherein the chemotherapeutic agent-resistant cancer is a hematological tumor;
[33] use of an HLA class I-recognizing antibody in the manufacture of a potentiator of a chemotherapeutic agent;
[34] use of [33], wherein the antibody is a low-molecular-weight antibody;
[35] use of [33] or [34], wherein a cancer to be treated with a chemotherapeutic agent is a chemotherapeutic agent-resistant cancer;
[36] use of [35], wherein the chemotherapeutic agent-resistant cancer is a hematological tumor;
[37] use of a chemotherapeutic agent and an HLA class I-recognizing antibody in the manufacture of a therapeutic pharmaceutical composition for cancer;
[38] use of [37], wherein the antibody is a low-molecular-weight antibody;
[39] use of [37] or [38], wherein the cancer is a chemotherapeutic agent-resistant cancer;
[40] use of [39], wherein the chemotherapeutic agent-resistant cancer is a hematological tumor;
[41] an HLA class I-recognizing antibody for use in a method of treating chemotherapeutic agent-resistant cancer;
[42] an HLA class I-recognizing antibody for use in a method of strengthening the effect of a therapeutic agent; and
[43] a therapeutic agent and an HLA class I-recognizing antibody for use in a method of treating cancer.

### Mode for Carrying Out the Invention

The present inventors discovered that anti-HLA class I antibodies have cytotoxic activity against chemotherapeutic agent-resistant cancers, thereby leading to the discovery of a potential treatment for chemotherapeutic agent-resistant cancer. The present invention is based on these findings.

The present invention relates to therapeutic agents for chemotherapeutic agent-resistant cancer, such agents including an HLA class I-recognizing antibody as an active ingredient.

In the context of the present invention, examples of HLA class I-recognizing antibodies include antibodies having biological functions and whose antigen is HLA class I.

In the context of the present invention, HLA refers to human leukocyte antigen. HLA molecules are categorized into class I and class II. Known examples of class I are HLA-A, B, C, E, F, G, H, J, and such. The antigens recognized by the antibodies of the present invention are not particularly limited, so long as they are molecules classified as HLA class I, preferably HLA-IA and more preferably the α2 domain of HLA class IA.

The origin of the antibodies of the present invention is not particularly limited. Accordingly, exemplary antibodies include those derived preferably from mammals, more preferably from humans.

Anti-HLA class I antibodies useful in the context of the present invention can be obtained as polyclonal or monoclonal antibodies using well known means. Mammal-derived monoclonal antibodies are particularly preferred anti-HLA class I antibodies for use in the context of the present invention. Mammal-derived monoclonal antibodies include those produced by hybridomas, and those produced by hosts transformed with an expression vector carrying an antibody gene using genetic engineering methods.

Examples of such antibodies include the C3B3 antibody (Japanese Patent Application No. 2006-193053), the 2D7 antibody (Kimura, et al., Biochem. Biophys. Res. Commun. (2004) 325: 1201-1209) and the like.

Basically, anti-HLA class I antibody-producing hybridomas can be prepared as follows, using conventional techniques. For example, HLA class I is used as a sensitizing antigen to perform immunizations according to conventional immunization methods. The obtained immunocytes are then fused with well-known parent cells according to conventional cell fusion methods. Monoclonal antibody-producing cells are then screened by ordinary screening methods. Antigens can be prepared by known methods, such as a method using baculoviruses (WO98/46777 and such). Alternatively, the genetic sequence of HLA class I is inserted into a known expression vector to transform it into suitable host cells, after which the HLA class I molecule of interest is purified by known methods from the host cells or from its culture supernatant. The resulting purified HLA class I protein can then be used as the sensitizing antigen. It is also possible to use a fusion protein, composed of HLA class I molecules fused with other proteins, as the sensitizing antigen.

Mammals that are immunized with a sensitizing antigen are not particularly limited, though it is preferable to take into consideration compatibility with the parent cells used for cell fusion. Thus, rodents such as mice, rats, or hamsters are generally selected.

Immunization of animals with a sensitizing antigen is performed according to known methods. For example, standard methods of delivering sensitizing antigen to mammals involve intraperitoneal or subcutaneous injection. More specifically, an appropriate amount of sensitizing antigen may be diluted and suspended with PBS (phosphate-buffered saline), physiological saline, or such. If desired, this may be mixed with an appropriate amount of a standard adjuvant, such as Freund's complete adjuvant, made into an emulsion, and then preferably administered to mammals several times every 4 to 21 days. An appropriate carrier may also be used during immunization with sensitizing antigens.

After such immunization, an increase in the level of desired antibodies in the serum is confirmed, immunocytes are obtained from the mammals and the immunocytes are subjected to cell fusion. Immunocytes that are preferably subjected to cell fusion are splenocytes in particular.

Regarding the other cells to be fused with the aforementioned immunocytes, mammalian myeloma cells used as parent cells include various known cell lines, such as P3X63Ag8.653 (Kearney, J. F. et al. J. Immunol. (1979) 123: 1548-1550), P3X63Ag8U.1 (Current Topics in Microbiology and Immunology (1978) 81: 1-7), NS-1 (Kohler. G. and Milstein, C. Eur. J. Immunol. (1976) 6: 511-519), MPC-11 (Margulies. D. H. et al., Cell (1976) 8: 405-415), SP2/0 (Shulman, M. et al., Nature (1978) 276: 269-270), FO (de St. Groth, S. F. et al., J. Immunol. Methods (1980) 35: 1-21), S194 (Trowbridge, I. S. J. Exp. Med. (1978) 148: 313-323), and R210 (Galfre, G. et al., Nature (1979) 277: 131-133).

In general, the above-mentioned immunocytes and myeloma cells may be fused according to standard methods, examples of which are described by Milstein *et al.* (Kohler. G. and Milstein, C., Methods Enzymol. (1981) 73: 3-46).

More specifically, the above-mentioned cell fusion is carried out, for example, in a standard nutrient medium in the presence of a cell fusion promoting agent. For example, polyethylene glycol (PEG), Sendai virus (HVJ), or such can be used as the fusion promoting agent. If desired, adjuvants such as dimethylsulfoxide can additionally be used to increase fusion efficiency.

Regarding the proportion of immunocytes and myeloma cells used, an example of a preferred ratio of myeloma cells to immunocytes is from 1:1 to 1:10. The medium used for the aforementioned cell fusion may be, for example, RPMI 1640 medium, MEM medium, and such, which are suitable for growth of the aforementioned myeloma cell lines, or other kinds of medium commonly used for such cell culturing. Serum supplements, such as fetal calf serum (FCS), may also be used in combination.

The cell fusion is carried out by thoroughly mixing prescribed amounts of the aforementioned immunocytes and myeloma cells in the above-mentioned medium, adding to the medium a solution of PEG preheated to about 37°C generally at a concentration of 30% to 60% (w/v), wherein the PEG has an average molecular weight of approximately 1,000-6,000, for example, and mixing them to form the desired fusion cells (hybridomas). A suitable medium is then successively added. Cell fusing agents and such that are undesirable for the growth of hybridomas can be removed by repeatedly removing the supernatant by centrifugation.

The hybridomas are selected by culturing them in a common selection medium such as HAT medium (a medium containing hypoxanthine, aminopterin, and thymidine). Culturing in the HAT medium is continued for a sufficient time, usually from a few days to a few weeks, to allow death of all cells but the target hybridomas (the non-fused cells). The usual limiting dilution method is then performed to screen and clone hybridomas producing the target antibodies.

The hybridomas prepared in this manner that produce the monoclonal antibodies may be subcultured in a common medium, and then stored for a long time in liquid nitrogen.

Monoclonal antibodies may be obtained from the hybridomas using conventional techniques; for example, the hybridomas are cultured according to standard methods and the antibodies may be obtained from the culture supernatants. Alternatively, the hybridomas are administered to a compatible mammal for proliferation and then the antibodies may be obtained from the ascites fluid. The former method is suitable for obtaining highly pure antibodies, while the latter method is more suitable for mass production of antibodies.

Recombinant antibodies produced by genetic engineering techniques can be used as the monoclonal antibodies of the present invention. They can be produced by cloning an antibody gene from a hybridoma; incorporating the antibody gene into an appropriate vector, and introducing the vector into a host (see, for example, Borrebaeck, C. A. K., and Larrick, J. W., THERAPEUTIC MONOCLONAL ANTIBODIES, Published in the United Kingdom by MACMILLAN PUBLISHERS LTD, 1990).

Specifically, mRNAs encoding the variable (V) regions of antibodies are isolated from cells producing the target antibodies, such as hybridomas, by known methods, for example, by preparing total RNAs using guanidine ultracentrifugation methods (Chirgwin, J. M. et al., Biochemistry (1979) 18: 5294-5299), AGPC methods (Chomczynski, P. et al., Anal. Biochem. (1987) 162: 156-159), or such, and then preparing mRNAs using an mRNA Purification Kit (manufactured by GE Healthcare Biosciences) or such. The mRNAs can also be prepared directly by using a QuickPrep mRNA Purification Kit (manufactured by GE Healthcare Biosciences).

The obtained mRNAs are used to synthesize cDNAs of the antibody V regions using reverse transcriptase. cDNAs may be synthesized using the AMV Reverse Transcriptase First-strand cDNA Synthesis Kit. Alternatively, cDNA may be synthesized and amplified following the 5'-RACE method (Frohman, M. A. et al., Proc. Natl. Acad. Sci. USA (1988) 85: 8998-9002; Belyavsky, A. et al., Nucleic Acids Res. (1989) 17: 2919-2932) that uses the 5'-Ampli FINDER RACE Kit (manufactured by Clontech), and PCR. A desired DNA fragment is purified from the obtained PCR products and linked to a vector DNA. From this, a recombinant vector is produced. The recombinant vector is then used to transform *E. coli* and such, and the desired recombinant vector is prepared from a selected colony. The nucleotide sequence of the desired DNA may then be identified through known methods, such as the deoxy method.

When DNAs encoding a V region of a desired antibody are obtained, they are linked to DNAs encoding a constant region (C region) of a desired antibody, and then these are incorporated into expression vectors. Alternatively, DNAs encoding an antibody V region can be incorporated into expression vectors that include DNAs of an antibody C region.

Antibodies to be used in the present invention are produced by incorporating the antibody gene into an expression vector so that it will be expressed under the control of an expression regulatory region, such as an enhancer or promoter, as described hereafter. Antibodies can be subsequently expressed by transforming host cells with this expression vector. Specific examples of antibodies to be used in the present invention include an antibody composed of heavy chain variable regions that include CDRs 1, 2, and 3, having the amino acid sequences of SEQ ID NOs: 7, 8, and 9, respectively, and an antibody composed of light chain variable regions that include CDRs 1, 2, and 3, having the amino acid sequences of SEQ ID NOs: 10, 11, and 12, respectively.

Examples of preferred antibodies of the present invention include antibodies having a heavy chain variable region of any one of (a) to (d) below:
(a) a heavy chain variable region having the amino acid sequence of SEQ ID NO: 2;
(b) a heavy chain variable region having an amino acid sequence that includes one or more amino acid substitutions, deletions, insertions, and/or additions in the amino acid sequence of SEQ ID NO: 2, and that is functionally equivalent to the heavy chain variable region of (a);
(c) a heavy chain variable region having an amino acid sequence encoded by a DNA that includes the nucleotide sequence of SEQ ID NO: 1; and
(d) a heavy chain variable region having an amino acid sequence encoded by a DNA that hybridizes under stringent conditions with a DNA that includes the nucleotide sequence of SEQ ID NO: 1.

Alternatively, exemplary antibodies of the present invention may have a light chain variable region of any one of (e) to (h) below:
(e) a light chain variable region having the amino acid sequence of SEQ ID NO: 4;
(f) a light chain variable region having an amino acid sequence that includes one or more amino acid substitutions, deletions, insertions, and/or additions in the amino acid sequence of SEQ ID NO: 4, and that is functionally equivalent to the light chain variable region of (e);
(g) a light chain variable region having an amino acid sequence encoded by a DNA that includes the nucleotide sequence of SEQ ID NO: 3; and
(h) a light chain variable region having an amino acid sequence encoded by a DNA that hybridizes under stringent conditions with a DNA that includes the nucleotide sequence of SEQ ID NO: 3.

Additional examples of antibodies of the present invention, composed of heavy chain variable regions and light chain variable regions, include antibodies having the amino acid sequence of any one of (a) to (d) below:
(a) the amino acid sequence of SEQ ID NO: 6;
(b) the amino acid sequence of SEQ ID NO: 6, including one or more amino acid substitutions, deletions, insertions, and/or additions therein;
(c) an amino acid sequence encoded by a DNA that includes the nucleotide sequence of SEQ ID NO: 5; and
(d) an amino acid sequence encoded by a DNA that hybridizes under stringent conditions with a DNA that includes the nucleotide sequence of SEQ ID NO: 5.

As noted above, the amino acid sequence of the heavy chain variable region or the light chain variable region may contain substitutions, deletions, additions, and/or insertions. It may further lack portions of heavy chain variable region and/or light chain variable region, or other polypeptides may be added, so long as the binding complex of heavy chain variable regions and light chain variable regions retains its antigen binding activity. Additionally, the variable region may be chimerized or humanized.

Herein, the term "functionally equivalent" means that the antibody of interest has an activity equivalent to the antibody composed of a heavy chain variable region that includes CDRs 1, 2, and 3, having the amino acid sequences of SEQ ID NOs: 7, 8, and 9, respectively, or a light chain variable region that includes CDRs 1, 2, and 3, having of the amino acid sequences of SEQ ID NOs: 10, 11, and 12, respectively (for example, HLA-A binding activity, cytotoxic activity, cell death-inducing activity, cell growth-suppressing activity, or such).

Methods for preparing polypeptides functionally equivalent to a certain polypeptide are well known to those skilled in the art, and include the introduction of mutations into polypeptides. For example, one skilled in the art can prepare an antibody functionally equivalent to an antibody of the present invention by introducing appropriate mutations into the antibody using site-directed mutagenesis (Hashimoto-Gotoh, T. et al., Gene (1995) 152: 271-275; Zoller MJ, and Smith M., Methods Enzymol. (1983) 100: 468-500; Kramer W. et al., Nucleic Acids Res. (1984) 12: 9441-9456; Kramer W, and Fritz HJ, Methods. Enzymol. (1987) 154: 350-367; Kunkel TA, Proc. Natl. Acad. Sci. USA (1985) 82: 488-492; Kunkel, Methods Enzymol. (1988) 85: 2763-2766). Amino acid mutations may also occur naturally. Therefore, the antibodies of the present invention also include antibodies functionally equivalent to the antibodies of the present invention, wherein the antibodies possess amino acid sequences that include one or more amino acid mutations as compared to the amino acid sequences of the antibodies of the present invention.

The number of amino acids that are mutated is not particularly limited, but is generally 30 amino acids or less, preferably 15 amino acids or less, and more preferably 5 amino acids or less (for example, 3 amino acids or less). Preferably, the mutated amino acids conserve the properties of the amino acid side chain from the amino acids that were mutated. Examples of amino acid side chain properties include: hydrophobic amino acids (A, I, L, M, F, P, W, Y, and V), hydrophilic amino acids (R, D, N, C, E, Q, G, H, K, S, and T), amino acids having the following side chains: aliphatic side chains (G, A, V, L, I, and P); hydroxyl-containing side chains (S, T, and Y); sulfur-containing side chains (C and M); carboxylic acid- and amide-containing side chains (D, N, E, and Q); basic side chains (R, K, and H); and aromatic ring-containing side chains (H, F, Y, and W) (amino acids are represented by one-letter codes in parentheses). Polypeptides having a modified amino acid sequence, in which one or more amino acid residues are deleted, added, and/or substituted with other amino acids, are known to retain their original biological activities (Mark, D. F. et al., Proc. Natl. Acad. Sci. USA (1984) 81: 5662-5666; Zoller, M. J. & Smith, M. Nucleic Acids Research (1982) 10: 6487-6500; Wang, A. et al., Science 224: 1431-1433; Dalbadie-McFarland, G. et al., Proc. Natl. Acad. Sci. USA (1982) 79: 6409-6413).

The antibodies of the present invention also include antibodies in which several amino acid residues have been added to an amino acid sequence of an antibody of the present invention. Fusion proteins, in which such antibodies are fused together with other peptides or proteins, are also included in the present invention. A fusion protein can be prepared by ligating a polynucleotide encoding an antibody of the present invention and a polynucleotide encoding another peptide or polypeptide such that the reading frames match, inserting this sequence into an expression vector, and expressing the fusion construct in a host. Techniques known to those skilled in the art are available for this purpose. Examples of peptides or polypeptides to be fused with an antibody of the present invention include FLAG (Hopp, T.P. et al., Biotechnology (1988) 6: 1204-1210), 6x His consisting of six His (histidine) residues, 10x His, Influenza hemagglutinin (HA), human c-myc fragment, VSV-GP fragment, p18HIV fragment, T7-tag, HSV-tag, E-tag, SV40T antigen fragment, lck tag, α-tubulin fragment, B-tag, Protein C fragment, and such. Examples of other polypeptides to be fused to the antibodies of the present invention include, GST (glutathione-S-transferase), HA (Influenza hemagglutinin), immunoglobulin constant region, β-galactosidase, MBP (maltose-binding protein), and such. Commercially available polynucleotides encoding these peptides or polypeptides can be fused with polynucleotides encoding the antibodies of the present invention. The fusion polypeptide can be prepared by expressing the fusion construct.

As described in detail below, the antibodies used in the present invention may differ in amino acid sequence, molecular weight, and isoelectric point, and may also differ in terms of the presence or absence of sugar chains and conformation, depending on the cell or host producing the antibody or purification method. However, so long as the obtained antibody is functionally equivalent to an antibody of the present invention, it can be used as the antibody of the present invention. For example, when an antibody of the present invention is expressed in a prokaryotic cell, such as *E*. *coli*, a methionine residue is added to the N terminus of the amino acid sequence of the original antibody. The antibodies used in the present invention will also include such antibodies.

In the present invention, genetically modified antibodies produced by incorporating an antibody gene into a suitable vector and introducing this vector into a host using genetic engineering techniques (for example, see Carl, A. K. Borrebaeck, James, W. Larrick, THERAPEUTIC MONOCLONAL ANTIBODIES, Published in the United Kingdom by MACMILLAN PUBLISHERS LTD, 1990) can be used. More specifically, when DNAs encoding heavy chain variable regions composed of CDRs 1, 2, and 3, having the amino acid sequences of SEQ ID NOs: 7, 8, and 9, respectively, or light chain variable regions composed of CDRs 1, 2, and 3, having the amino acid sequences of SEQ ID NOs: 10, 11, and 12, respectively, are obtained, they are linked to a DNA encoding a desired antibody constant region (C region), and then incorporated into an expression vector. Alternatively, a DNA encoding an antibody variable region can be incorporated into an expression vector that comprises a DNA of an antibody constant region. The DNA is incorporated into the expression vector such that it is expressed under the control of an expression regulatory region (enhancer or promoter). The antibody can then be expressed by transforming host cells with this expression vector.

Antibodies of the present invention also includes polynucleotides encoding the antibodies of the present invention, or polynucleotides that hybridize under stringent conditions to the polynucleotides of the present invention and encode antibodies having an activity equivalent to that of the antibodies of this invention. The polynucleotides of the present invention are polymers composed of multiple nucleic bases or base pairs of deoxyribonucleic acids (DNA) or ribonucleic acids (RNA), and are not particularly limited, as long as they encode the antibodies of the present invention. Polynucleotides of the present invention may also contain non-natural nucleotides. The polynucleotides of the present invention can be used to express antibodies using genetic engineering techniques. Furthermore, they can be used as probes in the screening of antibodies functionally equivalent to the antibodies of the present invention. Specifically, DNAs that hybridize under stringent conditions to the polynucleotides encoding the antibodies of the present invention, and encode antibodies having an activity equivalent to that of the antibodies of the present invention, can be obtained by techniques such as hybridization and gene amplification (for example, PCR), using a polynucleotide of the present invention or a portion thereof as a probe. Such DNAs are included in the polynucleotides of the present invention. Hybridization techniques are well known to those skilled in the art (Sambrook, J. et al., Molecular Cloning 2nd ed., 9.47-9.58, Cold Spring Harbor Lab. press, 1989).

Those skilled in the art can suitably select stringent hybridization conditions. As an example, in a hybridization solution containing 25% formamide, more stringently 50% formamide, and 4x SSC, 50 mM Hepes pH 7.0, 10x Denhardt's solution, and 20 µg/mL denatured salmon sperm DNA, pre-hybridization is performed overnight at 42°C, after which a labeled probe is added, and hybridization is performed by incubating overnight at 42°C. A subsequent wash can be performed using washing solution and temperature conditions of about "1x SSC, 0.1% SDS, 37°C", more stringent conditions are about "0.5x SSC, 0.1% SDS, 42°C", and even more stringent conditions are about "0.2x SSC, 0.1% SDS, 65°C". As hybridization washing conditions become more stringent, it is expected that DNA with higher homology to the probe sequence will be isolated. However, the above-mentioned combined conditions of SSC, SDS, and temperature are exemplary, and those skilled in the art can achieve stringencies similar to those mentioned above by appropriately combining the above-mentioned factors or other factors that determine hybridization stringency (for example, probe concentration, length of the probes, hybridization reaction time, and such).

An antibody encoded by a polynucleotide obtained by a hybridization technique and gene amplification technique, and which is functionally equivalent to an antibody of the present invention, generally has high homology to the amino acid sequence of the antibody of this invention. The antibodies of the present invention include antibodies that are functionally equivalent and have high amino acid sequence homology to the antibodies of the present invention. The term "high homology" generally means identity at the amino acid level of at least 50% or higher, preferably 75% or higher, more preferably 85% or higher, and still more preferably 95% or higher. Polypeptide homology can be determined by the algorithm described in Wilbur, W. J. and Lipman, D. J. Proc. Natl. Acad. Sci. USA (1983) 80: 726-730.

Preferred examples of antibodies used in the present invention also include antibodies encoded by the polynucleotides of (a) and (b):
(a) a polynucleotide having the nucleotide sequence of SEQ ID NO: 1, 3, or 5; or
(b) a polynucleotide that hybridizes under stringent conditions with the polynucleotide of (a) and encodes an antibody having an activity equivalent to the antibody of the present invention.

Alternatively, artificially modified genetically-recombinant antibodies, such as chimeric and humanized antibodies, may be used to reduce heterologous antigenicity against humans. These modified antibodies can be produced using known methods. A chimeric antibody is an antibody composed of the heavy and light chains variable regions of an antibody from a non-human mammal, such as a mouse, and the heavy and light chains constant regions of a human antibody. The chimeric antibody can be produced by linking a DNA encoding a mouse antibody variable region with a DNA encoding a human antibody constant region, incorporating this into an expression vector, and then introducing the vector.

The chimeric antibody can be produced by linking a DNA encoding the antibody V regions obtained as described above with a DNA encoding the C regions of the human antibody, incorporating this into an expression vector, and then introducing the vector into a host (see European Patent Application No. EP 125023 and International Patent Application WO 92/19759). Chimeric antibodies useful for the present invention can be obtained using this known method.

Humanized antibodies are also referred to as "reshaped human antibodies". Such humanized antibodies are obtained by grafting the complementarity determining region (CDR) of an antibody derived from a non-human mammal, for example, a mouse, to the CDR of a human antibody. Such general gene recombination procedures are also known.

For example, a DNA sequence designed to link a murine antibody CDR to the framework region (FR) of a human antibody is synthesized by PCR, using several oligonucleotides produced to contain overlapping portions in the terminal regions. The obtained DNA is linked to a DNA encoding a human antibody constant region, and incorporated into an expression vector. The antibody is produced by introducing this vector into a host (see European Patent Application No. EP 239400, and International Patent Application WO 96/02576).

The human antibody FR to be linked *via* CDR is selected such that the CDR forms a favorable antigen-binding site. In order for the CDR of the reshaped human antibody to form a suitable antigen-binding site, the amino acids in the framework region of the antibody variable region may be substituted as necessary (Sato, K. et al., Cancer Res. (1993) 53: 851-856).

Human antibody C regions are used in chimeric antibodies and humanized antibodies. An example of a human antibody C region is Cγ; thus, Cγ1, Cγ2, Cγ3, or Cγ4 may be used. In addition, to improve the stability of antibodies or the production thereof, the human antibody C-region may be modified.

A chimeric antibody includes the variable region of an antibody derived from a non-human mammal and the C-region derived from a human antibody. A humanized antibody is composed of the CDR of an antibody derived from a non-human mammal and a framework region and C region derived from a human antibody. Since the antigenicity of both these antibodies is low in human body, they are suitable as antibodies of the present invention.

Methods for obtaining human antibodies are also known. For example, human lymphocytes can be sensitized *in vitro* with a desired antigen, or with cells expressing a desired antigen, and the sensitized lymphocytes can be fused with human myeloma cells, such as U266, to obtain the desired human antibody with antigen-binding activity (see Japanese Patent Application Kokoku Publication No. (JP-B) Hei 1-59878 (examined, approved Japanese patent application published for opposition)). Further, a desired human antibody can be obtained by using a desired antigen to immunize transgenic animals that have a full repertoire of human antibody genes (see International Patent Application WO 93/12227, WO 92/03918, WO 94/02602, WO 94/25585, WO 96/34096, and WO 96/33735). Additional known techniques for obtaining human antibodies include panning using a human antibody library. For example, variable regions of human antibodies can be expressed as single-chain antibodies (scFvs) on the surface of phages using phage display methods, from which phages that bind to antigens can be selected. DNA sequences encoding the variable regions of human antibodies that bind to the antigens can be determined by analyzing the genes of the selected phages. By revealing the DNA sequences of the scFvs that bind to the antigens, appropriate expression vectors carrying the sequences can be produced to yield human antibodies. These methods are already known, and the following publications can be referred to: WO 92/01047, WO 92/20791, WO 93/06213, WO 93/11236, WO 93/19172, WO 95/01438, and WO 95/15388.

The antibody gene constructed as described above can be expressed using known methods. When mammalian cells are used, such an antibody gene can be expressed using a DNA in which a common useful promoter, the antibody gene to be expressed, and a poly A signal positioned downstream of the gene on the 3' side are operably linked, or using a vector carrying the DNA. An example of a promoter/enhancer is the human cytomegalovirus immediate early promoter/enhancer.

Furthermore, as a promoter/enhancer that can be used for the expression of an antibody of the present invention, viral promoter/enhancers of retroviruses, polyomaviruses, adenoviruses, simian virus 40 (SV40), or such, or mammalian cell-derived promoter/enhancers such as human elongation factor 1α (HEF1α) or such can be used.

Antibody expression can be easily carried out by following, for example, the method of Mulligan *et al.* (Mulligan, R. C. et al., Nature (1979) 277: 108-114), using the SV40 promoter/enhancer, or the method of Mizushima *et al.* (Mizushima, S. and Nagata, S. Nucleic Acids Res. (1990) 18: 5322), using the HEF1α promoter/enhancer.

In the case of *E. coli,* the antibody can be expressed by an operably linked common useful promoter, a signal sequence for antibody secretion, and an antibody gene to be expressed. Examples of promoters include a lacZ promoter and an araB promoter. A lacZ promoter can be used according to the method of Ward *et al.* (Ward, E. S. et al., Nature (1989) 341: 544-546; Ward, E. S. et al., FASEB J. (1992) 6: 2422-2427) and an araB promoter can be used according to the method of Better *et al.* (Better, M. et al., Science (1988) 240: 1041-1043).

When antibody production is carried out in the periplasm of *E. coli,* the pelB signal sequence (Lei, S. P. et al. J. Bacteriol. (1987) 169: 4379-4383) may be used as a signal sequence for antibody secretion. After antibodies produced in the periplasm are separated, the antibody structure is appropriately refolded and then used (see for example WO96/30394).

The replication origin may derive from SV40, polyoma viruses, adenoviruses, bovine papilloma viruses (BPV), and such. Furthermore, to increase the gene copy number in the host cells, the expression vector may contain, as a selection marker, an aminoglycoside phosphotransferase (APH) gene, thymidine kinase (TK) gene, *E. coli* xanthine guanine phosphoribosyl transferase (Ecogpt) gene, dihydrofolate reductase (dhfr) gene, and such.

Any production system may be used to produce the antibodies of the present invention. *In vitro* and *in vivo* production systems are available for antibody production systems. Production systems that use eukaryotic cells or prokaryotic cells are examples of *in vitro* production systems.

Production systems that use animal cells, plant cells, or fungal cells are available when using eukaryotic cells. Known animal cells include (1) mammalian cells, for example, CHO, COS, myeloma, BHK (baby hamster kidney), HeLa, Vero, and such, (2) amphibian cells such as *Xenopus laevis* oocytes, and (3) insect cells such as sf9, sf21, Tn5, and such. Known plant cells include *Nicotiana tabacum*-derived cells and these cells may be cultured as calluses. Known fungal cells include yeast, for example, the genus Saccharomyces, such as *Saccharomyces cerevisiae*; and filamentous fungi, for example, the genus Aspergillus such as *Aspergillus niger.*

Production systems that use bacterial cells are available when using prokaryotic cells. Examples of bacterial cells include *E*. *coil* and *Bacillus subtilis.*

Antibodies can be obtained by introducing the antibody gene of interest into these cells by transformation, then culturing these transformants *in vitro.* Transformants can be cultured using known methods. For example, DMEM, MEM, RPMI 1640, or IMDM may be used as a culture medium, and this may be used with serum supplements such as fetal calf serum (FCS). Furthermore, antibodies may be produced *in vivo* by transferring the antibody gene-introduced cells into the peritoneal cavity or such of the animals.

On the other hand, *in vivo* production systems include production systems using animals and production systems using plants. Mammals, insects and the like are used for production systems using animals.

Mammals such as goat, pig, sheep, mice, and cattle may be used (Vicki Glaser, SPECTRUM Biotechnology Applications, 1993). Alternatively, insects such as silkworms may be used. Tobacco, for example, can be used when using plants.

Antibody genes are introduced into such animals or plants, upon which the antibodies are produced in the body of the animals or plants and then recovered. For example, an antibody gene is prepared as a fusion gene by inserting the antibody gene into a gene encoding a protein that is specifically produced in milk, such as the goat β-casein gene. DNA fragments containing the fusion gene to which the antibody gene has been inserted are then injected into goat embryos, which are then introduced into female goats. The desired antibody can then be obtained from milk produced by the transgenic goats, which are born from the goats that received the embryos, or from their offspring. Hormones may be suitably administered to the transgenic goats to increase the production of milk containing the antibody of interest (Ebert, K.M. et al., Bio/Technology (1994) 12: 699-702).

When silkworm is used, baculoviruses carrying an antibody gene of interest is used to infect silkworms, whereupon the antibody of interest is obtained from their body fluids (Maeda, S. et al., Nature (1985) 315: 592-594). When tobacco is used, an antibody gene of interest is inserted into a plant expression vector, for example, pMON 530, and the vector may then be introduced into a bacterium, such as *Agrobacterium tumefaciens.* The bacteria are then used to infect tobacco, such as *Nicotiana tabacum,* whereupon the desired antibodies are obtained from the leaves of this tobacco (Julian K.-C. Ma et al., Eur. J. Immunol. (1994) 24: 131-138).

When producing an antibody using an *in vitro* or *in vivo* production system as described above, a DNA encoding the heavy chain (H chain) or the light chain (L chain) of the antibody can be each separately incorporated into a expression vector to simultaneously transform the host cell, or alternatively a DNA encoding the H chain and the L chain can be incorporated into a single expression vector to transform the host cell (see International Patent Application WO 94/11523).

Antibodies used in the present invention may be low-molecular-weight antibodies/minibodies. In the present invention, low-molecular-weight antibodies include antibody fragments derived from a parent antibody, i.e. a whole antibody (for example, whole IgG), in which part of the whole antibody is missing; the antibody fragment is not particularly limited so long as it has antigen-binding ability. Antibody fragments of the present invention are not particularly limited so long as they are part of a whole antibody. However, fragments containing heavy chain variable regions (VH) or light chain variable regions (VL) are preferred, and fragments containing both VH and VL are particularly preferred. Specific examples of antibody fragments include Fab, Fab', F(ab')2, Fv, scFv (single chain Fv), sc(Fv)₂ and such, but are preferably diabodies (Huston, J. S. et al., Proc. Natl. Acad. Sci. USA (1988) 85: 5879-5883; Plickthun "The Pharmacology of Monoclonal Antibodies" Vol.113, Resenburg and Moore ed., Springer Verlag, New York, pp.269-315, (1994)). Such antibody fragments can be obtained by treating an antibody with enzymes such as papain or pepsin to produce antibody fragments, or by constructing genes encoding such antibody fragments, introducing them into an expression vector, and then expressing this in an appropriate host cell (see for example, Co, M. S. et al., J. Immunol. (1994) 152: 2968-2976; Better, M. and Horwitz, A. H., Methods Enzymol. (1989) 178: 476-496; Pluckthun, A. and Skerra, A., Methods Enzymol. (1989) 178: 497-515; Lamoyi, E., Methods Enzymol. (1986) 121: 652-663; Rousseaux, J. et al., Methods Enzymol. (1986) 121: 663-669; Bird, R. E. and Walker, B. W., Trends Biotechnol. (1991) 9: 132-137).

The molecular weight of the low-molecular-weight antibody of the present invention is preferably smaller than that of the whole antibody; however, multimers such as dimers, trimers, and tetramers may be formed, such that the molecular weight can be larger than the molecular weight of the whole antibody.

Low-molecular-weight antibodies of the present invention are preferably antibodies composed of two or more VH and two or more VL of an antibody, and these variable regions are linked directly or indirectly through linkers or such. The linkages may be covalent bonds, non-covalent bonds, or both covalent and non-covalent bonds. A more preferred low-molecular-weight antibody is an antibody composed of two or more VH-VL pairs formed by linking VH and VL with a non-covalent bond. In this case, a low-molecular-weight antibody having a shorter distance between one VH-VL pair and the other VH-VL pair than the distance in the whole antibody is preferred.

In the present invention, scFv is obtained by ligating an antibody H chain V region with an antibody L chain V region. In this scFv, the H chain V region and L chain V region are ligated *via* a linker, preferably *via* a peptide linker (Huston, J. S. et al., Proc. Natl. Acad. Sci. USA (1988) 85: 5879-5883). The H-chain V-region and L-chain V-region in scFv may be derived from any of the antibodies described herein. For example, any single-chain peptides composed of 12 to 19 amino acid residues may be used as a peptide linker for ligating the V regions.

DNAs encoding scFv can be obtained by using as a template, DNAs encoding the antibody H chain or H chain V region and the antibody L chain or L chain V region mentioned above, and, from among those sequences, amplifying a DNA portion that encodes the desired amino acid sequence by PCR using a primer pair that defines its two ends; and then carrying out a subsequent amplification using a combination of a DNA encoding the peptide linker portion, and the primer pair that defines both ends of the linker DNA to be ligated to the H chain and the L chain, respectively.

Once a DNA encoding an scFv is constructed, an expression vector containing the DNA, and a host transformed with the expression vector can be obtained according to conventional methods. Furthermore, scFvs can be obtained using these hosts according to conventional methods.

These antibody fragments can be produced in hosts by obtaining their genes and expressing them in a manner similar to that described above. These antibody fragments are included as "antibodies" of the present invention.

Low-molecular-weight antibodies that are particularly preferred in the present invention are diabodies. Diabodies are dimers formed by linking two fragments (such as scFvs; hereinafter referred to as diabody-constituting fragments), in which a variable region is linked to another variable region *via* a linker or such. Ordinarily, diabodies composed of two VLs and two VHs (P. Holliger et al., Proc. Natl. Acad. Sci. USA (1993) 90: 6444-6448; EP 404097; WO 93/11161; Johnson et al., Method in Enzymology (1991) 203: 88-98; Holliger et al., Protein Engineering (1996) 9: 299-305; Perisic et al., Structure (1994) 2: 1217-1226; John et al., Protein Engineering (1999) 12 (7): 597-604; Holliger et al,. Proc. Natl. Acad. Sci. USA (1993) 90: 6444-6448; Atwell et al., Mol. Immunol. (1996) 33: 1301-1312). Bonds between diabody-constituting fragments may be non-covalent or covalent bonds, but are preferably non-covalent bonds.

Alternatively, diabody-constituting fragments may be linked to each other by a linker and such to form a single-chain diabody (sc diabody). In such cases, linking diabody-constituting fragments using a long linker of about 20 amino acids allows the diabody-constituting fragments on the same chain to form a dimer with each other *via* non-covalent bonds.

Diabody-constituting fragments include those with linked VL-VH, VL-VL, and VH-VH, more preferably those with linked VH-VL. In diabody-constituting fragments, the linker used to link a variable region to a variable region is not particularly limited, but is preferably short enough to prevent non-covalent bonding between variable regions in the same fragment. The length of such a linker can be suitably determined by those skilled in the art, and is ordinarily 2 to 14 amino acids, preferably 3 to 9 amino acids, and more preferably 4 to 6 amino acids. In this case, linkers between VL and VH encoded on a same fragment are short, and thus VL and VH on a same strand do not form a non-covalent bond; therefore a single-chain V region fragment will not be formed. Rather, a fragment forms a dimer with another fragment *via* non-covalent bonding. Furthermore, according to the same principle in diabody construction, three or more diabody-constituting fragments may be linked to form multimeric antibodies such as trimers and tetramers.

Exemplary diabodies of the present invention include, but are not limited to, a diabody having the amino acid sequence of SEQ ID NO: 6; a diabody that is functionally equivalent to a diabody comprising the sequence of SEQ ID NO: 6 and has an amino acid sequence that includes one or more amino acid mutations (substitutions, deletions, insertions, and/or additions) in the amino acid sequence of SEQ ID NO: 6; a diabody having the amino acid sequences of the CDRs (or variable regions) of SEQ ID NO: 2 and SEQ ID NO: 4; and a diabody that is functionally equivalent to a diabody having the amino acid sequences of the CDRs (or variable regions) of SEQ ID NO: 2 and SEQ ID NO: 4, and has an amino acid sequence that includes one or more amino acid mutations (substitutions, deletions, insertions, and/or additions) in the amino acid sequences of the CDRs (or variable regions) of SEQ ID NO: 2 and SEQ ID NO: 4.

Herein, the phrase "functionally equivalent" means that the diabody of interest has an equivalent activity to that of a diabody having the sequence of SEQ ID NO: 6, or that of a diabody having the sequences of the CDRs (or variable regions) of SEQ ID NO: 2 and SEQ ID NO: 4 (for example, HLA-A binding activity, cytotoxic activity, cell death-inducing activity, and cell growth-suppressing activity).

The number of amino acids to be mutated is not particularly limited; however, it is usually 30 amino acids or less, preferably 15 amino acids or less, and more preferably 5 amino acids or less (for example, 3 amino acids or less).

Furthermore, a diabody having the amino acid sequence of SEQ ID NO: 6, or a diabody having the sequences of the CDRs (or variable regions) of SEQ ID NO: 2 and SEQ ID NO: 4 may be humanized or chimerized to reduce heterologous antigenicity against human.

In the amino acid sequence of SEQ ID NO: 2, amino acids 1 to 125 correspond to the variable region, amino acids 31 to 35 correspond to CDR1 (SEQ ID NO: 7), amino acids 50 to 66 correspond to CDR2 (SEQ ID NO: 8), and amino acids 99 to 114 correspond to CDR3 (SEQ ID NO: 9). In the amino acid sequence of SEQ ID NO: 4, amino acids 1 to 107 correspond to the variable region, amino acids 24 to 34 correspond to CDR1 (SEQ ID NO: 10), amino acids 50 to 56 correspond to CDR2 (SEQ ID NO: 11), and amino acids 89 to 97 correspond to CDR3 (SEQ ID NO: 12).

In the present invention, the HLA-recognizing low-molecular-weight antibodies specifically bind to HLA, and are not particularly limited, so long as they have biological activities. The low-molecular-weight antibodies of the present invention can be prepared by methods well known to those skilled in the art. For example, as described in the Examples, the antibodies can be prepared based on the sequence of an HLA-recognizing antibody (particularly, sequences of the variable regions and CDRs), using genetic engineering techniques known to those skilled in the art.

A well-known antibody sequence can be used as the HLA-recognizing antibody sequence; alternatively, an anti-HLA antibody can be prepared by a method well known to those skilled in the art using HLA as an antigen, and then this antibody sequence can be obtained and used.

In the present invention, the phrase "anti-HLA class I antibody activity" refers to a biological action resulting from antigen-antibody binding. Examples of such biological actions include cytotoxic actions, anti-tumor actions, and such, though they are not limited thereto. More specific examples include cell death-inducing actions, apoptosis-inducing actions, cell growth-suppressing actions, cell differentiation-suppressing actions, cell division-suppressing actions, cell growth-inducing actions, cell differentiation-inducing actions, cell division-inducing actions, and cell cycle-regulating actions. Cell death-inducing actions and cell growth-suppressing actions are preferred.

Target cells of the above-mentioned actions, such as cell death-inducing actions and cell growth-suppressing actions, are not particularly limited, though hematopoietic cells and non-adherent cells are preferred. Specific examples of hematopoietic cells include lymphocytes (B cells, T cells), neutrophils, eosinophils, basophils, monocytes (preferably activated peripheral blood mononuclear cells (PBMC)), and myeloma cells, while lymphocytes (B cells, T cells), and myeloma cells are preferred, and T cells or B cells (in particular, activated B cells or T cells) are most preferable. The phrase "non-adherent cells" refer to cells that, when cultured, grow in a non-adherent state without adhering to the surface of culturing vessels made of glass, plastic or the like. On the other hand, the phrase "adherent cells" refer to cells that, when cultured, adhere to the surface of culturing vessels made of glass, plastic or the like.

Generally, to exhibit enhanced cell death-inducing activity, a full length anti-HLA antibody can be crosslinked with an anti-IgG antibody or such, and the crosslinking can be accomplished by methods known to those skilled in the art.

In the present invention, administration of the above-mentioned HLA-recognizing antibody can be used to treat or prevent diseases such as tumors, including hematological tumors (specific examples include leukemia; myelodysplastic syndrome; malignant lymphoma; Burkitt's lymphoma; chronic myeloid leukemia; acute myeloid leukemia; plasmacytic disorders such as myeloma, multiple myeloma, and macroglobulinemia; and myeloproliferative diseases such as polycythemia vera, essential thrombocythemia, and idiopathic myelofibrosis; and such), and autoimmune diseases (specific examples include rheumatism, autoimmune hepatitis, autoimmune thyroiditis, autoimmune bullosis, autoimmune adrenocortical disease, autoimmune hemolytic anemia, autoimmune thrombycytopenic purpura, autoimmune atrophic gastritis, autoimmune neutropenia, autoimmune orchitis, autoimmune encephalomyelitis, autoimmune receptor disease, autoimmune infertility, Crohn's disease, systemic lupus erythematosus, multiple sclerosis, Basedow's disease, juvenile diabetes, Addison's disease, myasthenia gravis, lens-induced uveitis, psoriasis, and Behchet's disease). Furthermore, given the excellent stability of the antibodies of present invention *in vivo*, they are expected to be particularly efficacious when administered to living subjects.

Therapeutic agents for chemotherapeutic agent-resistant cancer of the present invention, including an HLA class I-recognizing antibody as an active ingredient, can be used to treat chemotherapeutic agent-resistant cancers.

Cancer chemotherapy refers to the use of chemotherapeutic agents to treat cancer. Responsiveness to chemotherapy differs depending on the case but generally, pharmaceutical agents which their function are based on a number of different mechanisms are administered in combination expecting for a synergistic effect and minimized side-effect.

Cancers resistant to chemotherapeutic agents include those having natural resistance, in which effects of chemotherapeutic agents cannot be observed from the beginning of the treatment, and those with acquired resistance, in which chemotherapeutic agents effective in the beginning lose their effectiveness as the treatment processes, thereby leading to recurrence of cancer. The chemotherapeutic agent-resistant cancers for which therapeutic agents of the present invention are used include both those having natural resistance and those acquired resistance, though cancers with acquired resistance are preferred. Characteristics of cancers with acquired resistance against chemotherapeutic agents include for example, excessive expression of drug-metabolizing enzymes or mutations in proteins targeting pharmaceutical agents, decreased uptake of pharmaceutical agents into cells, increased transport of pharmaceutical agents to the cell exterior, and such but are not limited thereto. A specific example of a protein involved in the transportation of pharmaceutical agents to the cell exterior is MDR1 (P glycoprotein).

Therapeutic agents of the present invention are used, though not limited, to the following cancers. Preferred examples include tumors, such as hematological tumors, and more specifically, resistant cancers such as leukemia, myelodysplastic syndrome, malignant lymphoma, Burkitt's lymphoma, chronic myeloid leukemia, acute myeloid leukemia, plasmacytic disorders (myeloma, multiple myeloma, macroglobulinemia), and such.

As used herein, the term "chemotherapeutic agents" includes alkylating agents, antimetabolites, natural products, platinum complexes, and other pharmaceutical agents. Examples of alkylating agents include nitrogen mustard, ethylenimines, methylmelamines, alkyl sulfonates, nitrosoureas, and triazens. Examples of nitrogen mustards include mechlorethamine, cyclophosphamide, ifosfamide, melphalan, and chlorambucil. Examples of ethylenimines and methylmelamines include hexamethylmelamine and thiotepa. An example of alkyl sulfonates includes busulfan. Examples of nitrosoureas include carmustine (BCNU), lomustine (CCNU), semustine (methyl-CCNU), and streptozocin. An example of triazens includes dacarbazine (DTIC). Examples of antimetabolites include folic acid analogs, pyrimidine analogs, and purine analogs. An example of a folic acid analog is methotrexate. Examples of pyrimidine analogs include, fluorouracil (5-FU), doxifluridine (5'-DFUR; product name: Furtulon), capecitabine (product name: Xeloda), floxuridine (FudR), and cytarabine. Examples of purine analogs include mercaptopurine (6-MP), thioguanine (TG), and pentostatin. Examples of natural products include vinca alkaloids, epipodophyllotoxins, and antibiotics. Examples of vinca alkaloids include vinblastine (VLB) and vincristine (VCR). Examples of epipodophyllotoxins include etoposide and teniposide. Examples of antibiotics include dactinomycin (actinomycin D), daunorubicin, doxorubicin, bleomycin, plicamycin, and mitomycin. Platinum complexes refer to platinum-coordinated complexes and examples include cisplatin (CDDP) and carboplatin. Examples of other pharmaceutical agents include taxoids such as paclitaxel and docetaxel, anthracenediones such as mitoxantrone, substituted ureas such as hydroxyurea, methylhydrazines such as procarbazine hydrochloride (product name: Natulan), and vitamin A metabolite such as tretinoin (product name: Vesanoid).

Anti-HLA class I antibodies of the present invention can be used as potentiators of chemotherapeutic agents.

In the present invention, the term "potentiators" refers to pharmaceutical agents having the ability to strengthen specific actions of other pharmaceutical agents when used for the same subject as the other pharmaceutical agent. Therefore, if a pharmaceutical agent to be examined has a function to strengthen the cytotoxic actions, cell death-inducing actions, or cell growth-suppressing actions when used for the same subject as the anticancer agent (chemotherapeutic agent), this pharmaceutical agent is deemed a "potentiator". Pharmaceutical agents that enhance anticancer activity are used on the same patients as those who are administered anticancer agents to thereby provide a synergistic effect or a distinct qualitative therapeutic effect as compared to the therapeutic effects observed when using anticancer agents alone.

Furthermore, anti-HLA class I antibodies of the present invention can be used in combination with the above-mentioned chemotherapeutic agents.

In the present invention, combined use of anti-HLA class I antibodies with chemotherapeutic agents means that an anti-HLA class I antibody and a chemotherapeutic agent are administered or used (hereinafter, simply referred to as "administered") together; however, there is no limitation on the order of administration or the interval of administration. Furthermore, an anti-HLA class I antibody of the present invention and a chemotherapeutic agent may be combinely used in the form of kits. Furthermore, when using the anti-HLA class I antibody of the present invention with the chemotherapeutic agent in combination, the respective doses may be reduced, if desired, as compared to when each of them is used alone.

The order of administration of an anti-HLA class I antibody of the present invention and a chemotherapeutic agent may be any of administering the chemotherapeutic agent first and then the anti-HLA class I antibody, administering the chemotherapeutic agent and the anti-HLA class I antibody simultaneously, or administering the anti-HLA class I antibody first and then the chemotherapeutic agent, but administering the anti-HLA class I antibody first and then the chemotherapeutic agent or administering the chemotherapeutic agent and the anti-HLA class I antibody simultaneously is preferred, and administering the anti-HLA class I antibody first and then the chemotherapeutic agent is even more preferred.

When administering the anti-HLA class I antibody first and then the chemotherapeutic agent, the interval between administration of the anti-HLA class I antibody and the chemotherapeutic agent is not particularly limited, and the interval can be determined by taking factors such as the administration route and dosage form in consideration. For example, the interval between administrations is ordinarily 0 to 72 hours, preferably 0 to 24 hours, more preferably 0 to 12 hours, and even more preferably 0 to 6 hours.

An anti-HLA class I antibody together with a chemotherapeutic agent can be made into a single pharmaceutical composition. Furthermore, an anti-HLA class I antibody can be made into a pharmaceutical composition which is used in combination with a chemotherapeutic agent. That is, an anti-HLA class I antibody can be used for the production of "a pharmaceutical composition composed of an anti-HLA class I antibody and a pharmaceutically acceptable carrier, in which the composition is used in combination with a chemotherapeutic agent". In addition, a chemotherapeutic agent can be made into a pharmaceutical composition which is used in combination with an anti-HLA class I antibody. More specifically, a chemotherapeutic agent can be used for the production of "a pharmaceutical composition composed of a chemotherapeutic agent and a pharmaceutically acceptable carrier, in which the composition is used in combination with an anti-HLA class I antibody".

The pharmaceutical compositions of the present invention can also be used as cell death-inducing agents or therapeutic agents for cancer. Therefore, the present invention provides cell death-inducing agents and therapeutic agents for cancer, such agents including a chemotherapeutic agent and an HLA class I-recognizing antibody as active ingredients. The present invention also provides cell death-inducing agents and therapeutic agents for cancer, such agents including an HLA class I-recognizing antibody as an active ingredient, wherein the agents are used in combination with a chemotherapeutic agent.

Subjects to which pharmaceutical agents of the present invention are administered are mammals. The mammals are preferably humans.

The pharmaceutical agents of the present invention can be administered in the form of a pharmaceutical, and can be administered orally or parenterally and systemically or topically. For example, intravenous injection such as drip infusion, intramuscular injection, intraperitoneal injection, subcutaneous injection, suppository, colonic infusion, or oral enteric coating agent may be selected, and a suitable administration method can be selected according to the age and symptoms of the patient. The effective dose can be selected from the range of 0.01 mg to 100 mg per kg body weight in each administration. Alternatively, the dosage can be selected from 1-1,000 mg per patient, or preferably 5-50 mg per patient. For example, preferred dose and method of administration refer to an effective dose, which is an amount that causes free antibodies to be present in the blood, and specific examples include administration methods such as administering 0.5 mg to 40 mg per month (four weeks) per kg body weight, which is preferably one mg to 20 mg in one to several doses, for example, by methods including intravenous injection such as drip infusion or subcutaneous injection following an administration schedule of twice/week, once/week, once/two weeks, once/four weeks, or such. The administration schedule can be adjusted by extending the administration interval from twice/week or once/week to once/two weeks, once/three weeks, or once/four weeks by observing post-administration condition and changes in blood test values.

Pharmaceutically acceptable carriers, such as preservatives and stabilizers, can be added to the pharmaceutical agents of the present invention. The term "pharmaceutically acceptable carrier" refers to a carrier that itself may be a material that has or does not have the above-described cytotoxic activity; the carrier is a material that can be administered together with the above-mentioned pharmaceutical agent. It may further be a material that does not have the cytotoxic activity, or a material that has a synergistic or additive stabilizing effect when used in combination with an anti-HLA class I antibody.

Examples of pharmaceutically acceptable materials include sterilized water, physiological saline, stabilizers, excipients, buffers, preservatives, surfactants, chelating agents (for example, EDTA), and binders and the like.

In the present invention, examples of surfactants include non-ionic surfactants. Typical examples include, sorbitan fatty acid esters such as sorbitan monocaprilate, sorbitan monolaurate, or sorbitan monopalmitate; glycerol fatty acid esters such as glycerol monocaprilate, glycerol monomyristate, or glycerol monostearate; polyglycerol esters of fatty acids such as decaglyceryl monostearate, decaglyceryl distearate, or decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, or polyoxyethylene sorbitan tristearate; polyoxyethylene sorbitol fatty acid esters such as polyoxyethylene sorbitol tetrastearate, polyoxyethylene sorbitol tetraoleate; polyoxyethylene glycerol fatty acid esters such as polyoxyethylene glyceryl monostearate; polyethylene glycol fatty acid esters such as polyethylene glycol distearate; polyoxyethylene alkyl ether such as polyoxyethylene lauyl ether; polyoxyethylene polyoxypropylene alkyl ether such as polyoxyethylene polyoxypropylene glycol, polyoxyethylene polyoxypropylene propylether, or polyoxyethylene polyoxypropylene cetyl ether; polyoxyethylene alkylphenyl ether such as polyoxyethylene nonylphenyl ether; polyoxyethylene hardened castor oils such as polyoxyethylene castor oil, or polyoxyethylene hardened castor oil (polyoxyethylene hydrogenated castor oil); polyoxyethylene beeswax derivatives such as polyoxyethylene sorbitol beeswax; polyoxyethylene lanolin derivatives such as polyoxyethylene lanolin; and polyoxyethylene fatty acid amide with HLB 6 to 18 such as polyoxyethylene stearylamide.

Anionic surfactants can also be listed as surfactants. Typical examples of anionic surfactants may include alkyl sulfate salts having an alkyl group of 10 to 18 carbon atoms, such as sodium cetyl sulfate, sodium lauryl sulfate, or sodium oleyl sulfate; polyoxyethylene alkylether sulfate salts whose average mole of ethyleneoxide added is two to four and the number of carbon atoms in the alkyl group is 10 to 18, such as sodium polyoxyethylene lauryl sulfate; alkyl sulfosuccinate ester salts of 8 to 18 carbon atoms in the alkyl group, such as sodium lauryl sulfosuccinate ester; naturally-occurring surfactants such as lecithin or glycerol lipid phosphate; sphingophospholipids such as sphingomyelin; and sucrose fatty acid esters of 12 to 18 carbon atoms in the fatty acid.

One or a combination of two or more of these surfactants can be added to the pharmaceutical agents of the present invention. A preferred surfactant to be used in the formulation of the present invention is a polyoxyethylene sorbitan fatty acid ester, such as Polysorbate 20, 40, 60, 80, or such, wherein Polysorbate 20 and 80 are particularly preferred. Polyoxyethylene polyoxypropylene glycol represented by Poloxamer (for example, Pluronic F-68 ®) is also preferred.

The amount of surfactant to be added differs depending on the type of surfactant used, but for Polysorbate 20 or Polysorbate 80, it is generally 0.001-100 mg/mL, preferably 0.003-50 mg/mL, and more preferably 0.005-2 mg/mL.

Examples of buffers in the present invention include such as phosphoric acid, citric acid buffer, acetic acid, malic acid, tartaric acid, succinic acid, lactic acid, calcium phosphate, gluconic acid, caprylic acid, deoxycholic acid, salicylic acid, triethanolamine, fumaric acid, other organic acids, carbonic acid buffer, Tris buffer, histidine buffer, imidazole buffer and the like.

Solution formulations can be prepared by dissolving into aqueous buffers that are known in the field of solution formulation. The buffer concentration is generally 1-500 mM, preferably 5-100 mM, and even more preferably 10-20 mM.

The therapeutic agents for chemotherapeutic agent-resistant cancer of the present invention may further include other low-molecular-weight polypeptides, proteins such as serum albumin, gelatin, and immunoglobulin, amino acids, sugars and carbohydrates such as polysaccharides and monosaccharides, and sugar alcohols.

Examples of amino acids in the present invention include basic amino acids such as arginine, lysine, histidine, and omithine, and inorganic salts of these amino acids (preferably in the form of chloride salts or phosphate salts, or more specifically amino-acid phosphates). When using free amino acids, the pH is adjusted to a preferred value by adding a suitable physiologically acceptable buffer, such as inorganic acids, particularly hydrochloric acid, phosphoric acid, sulfuric acid, acetic acid, formic acid, or a salt thereof. In such cases, the use of a phosphoric acid salt is particularly useful because a particularly stable freeze-dried product can be obtained. It is particularly advantageous when the preparation does not substantially contain an organic acid such as malic acid, tartaric acid, citric acid, succinic acid, or fumaric acid, or when a corresponding anion (maleate ion, tartarate, ion, citrate ion, succinate ion, fumarate ion, or such) is not present. Preferred amino acids are arginine, lysine, histidine, or ornithine. Furthermore, acidic amino acids such as glutamic acid and aspartic acid, and salts thereof (preferably sodium salts); neutral amino acids such as isoleucine, leucine, glycine, serine, threonine, valine, methionine, cysteine, or alanine; or aromatic amino acids such as phenylalanine, tyrosine, tryptophan, or its derivative, *N*-acetyltryptophan can be used.

Examples of sugars and carbohydrates, such as polysaccharides and monosaccharides, in the present invention include dextran, glucose, fructose, lactose, xylose, mannose, maltose, sucrose, trehalose, and raffinose.

Examples of sugar alcohols in the present invention include mannitol, sorbitol, inositol, and such.

When the pharmaceutical agent of the present invention is used as an aqueous solution used for injection, the solution can be mixed with, for example, physiological saline and isotonic solutions that include glucose or other adjunctive agents such as D-sorbitol, D-mannose, D-mannitol, and sodium chloride. The solution may also be combined with appropriate solubilizing agents, such as alcohol (for example, ethanol), polyalcohol (for example, propylene glycol or PEG), or non-ionic surfactant (for example, polysorbate 80 or HCO-50).

If desired, diluents, solubilizing agents, pH-adjusting agents, soothing agents, sulfur-containing reducing agents, and antioxidants may be included.

Examples of sulfur-containing reducing agents in the present invention include *N*-acetyl cysteine, *N*-acetyl homocysteine, thioctic acid, thiodiglycol, thioethanolamine, thioglycerol, thiosorbitol, thioglycolic acid, and salts thereof, sodium thiosulfate, glutathione, and compounds carrying a sulfhydryl group such as thioalkanoic acid of one to seven carbon atoms.

Examples of antioxidants in the present invention include erythorbic acid, dibutylhydroxytoluene, butylhydroxyanisole, α-tocopherol, tocopherol acetate, L-ascorbic acid and salts thereof, L-ascorbyl palmitate, L-ascorbyl stearate, sodium bisulfite, sodium sulfite, triamyl gallate, propyl gallate, and chelating agents such as ethylenediamine tetraacetic acid disodium (EDTA), sodium pyrophosphate, and sodium metaphosphate.

If necessary, the pharmaceutical agents can be contained within microcapsules (microcapsules made of hydroxymethylcellulose, gelatin, poly[methyl methacrylate], or such), or made into colloidal drug delivery systems (such as liposomes, albumin microspheres, microemulsion, nanoparticles, and nanocapsules) (see for example, "Remington's Pharmaceutical Science 16th edition", Oslo Ed., 1980). Methods for preparing the pharmaceutical agents as controlled-release pharmaceutical agents are also well known, and such methods may be applied to the present invention (Langer et al., J. Biomed. Mater. Res. (1981) 15: 167-277; Langer, Chem. Tech. (1982) 12: 98-105; U.S. Patent No. 3,773,919; European Patent (EP) Patent Application No. 58,481; Sidman et al., Biopolymers (1983) 22: 547-556; EP 133,988).

Pharmaceutically acceptable carriers used are suitably selected from those mentioned above or combinations thereof according to the dosage form, but are not limited thereto.

The present invention relates to methods for treating chemotherapeutic agent-resistant cancers which include the step of administering to a subject an HLA class I-recognizing antibody. Furthermore, the present invention relates to methods for inducing cell death and methods for treating cancer, in which a chemotherapeutic agent and an HLA class 1-recognizing antibody are used in combination.

In the present invention, the term "subject" refers to an organism or a part of the body of the organism, to which a therapeutic agent for chemotherapeutic agent-resistant cancer of the present invention is administered. The organism is not particularly limited, and includes animals (for example, humans, domesticated animals, and wild animals). There is no particular limitation on the above-mentioned "part of the body of the organism".

In the present invention, the phrase "to administer" includes administering orally or parenterally. Oral administration includes administration in the form of oral agents, and the dosage form for oral agents can be selected from granules, powders, tablets, capsules, dissolved agents, emulsion, suspension, and such.

Parenteral administration includes administration in the injectable form, examples of which include intravenous injection, subcutaneous injection, intramuscular injection, and intraperitoneal injection. Furthermore, effects of the methods of the present invention can be accomplished by introducing a gene composed of an oligonucleotide to be administered into a living organism using gene therapy techniques. The pharmaceutical agents of the present invention can be administered locally to a region to be treated. For example, the agents can be administered by local infusion or by the use of a catheter during surgery, or by targeted gene delivery of DNA encoding a peptide of the present invention.

A pharmaceutical agent of the present invention may be administered to a subject once or in a successive manner.

When administering a pharmaceutical agent of the present invention to a part of an organism that is removed or discharged from the organism, the pharmaceutical agent can be in "contact" with the part of the organism.

In the present invention, the "contact" is made according to the condition of the organism. For example, a pharmaceutical agent of the present invention can be sprayed on to a part of an organism or added to a homogenate of a part of an organism, but are not limited thereto. When a part of an organism is cultured cells, the above-mentioned "contact" can be carried out by adding a pharmaceutical agent of the present invention to a culture medium of the cells, or by introducing a DNA composed of an oligonucleotide of the present invention to cells that constitute a portion of the organism.

All prior art reference cited herein are incorporated by reference in their entirety.

### Brief Description of the Drawings

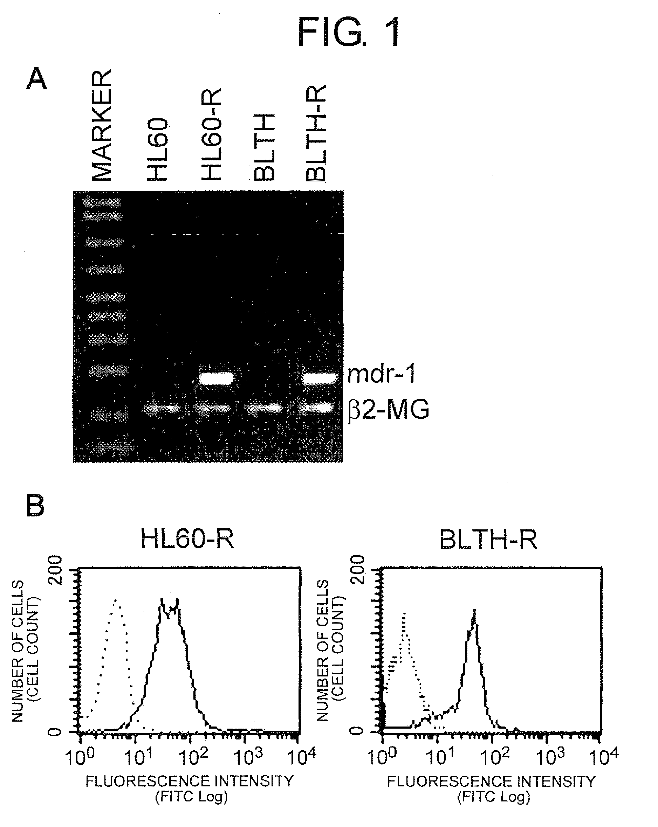

Fig. 1 includes a photograph and graphs indicating the expression of mdr1 mRNA and the MDR1 protein in chemotherapeutic agent-resistant cell lines. (A) is a photograph depicting the amount of mRNA expression as determined by RT-PCR. (B) depicts the amount of MDR1 expression on the cell surface as determined by flow cytometry using anti-MDR1 antibody and FITC-labeled goat anti-mouse IgG antibody. The dotted lines and the solid lines indicate the measured results obtained using a control antibody and the anti-MDR1 antibody, respectively.

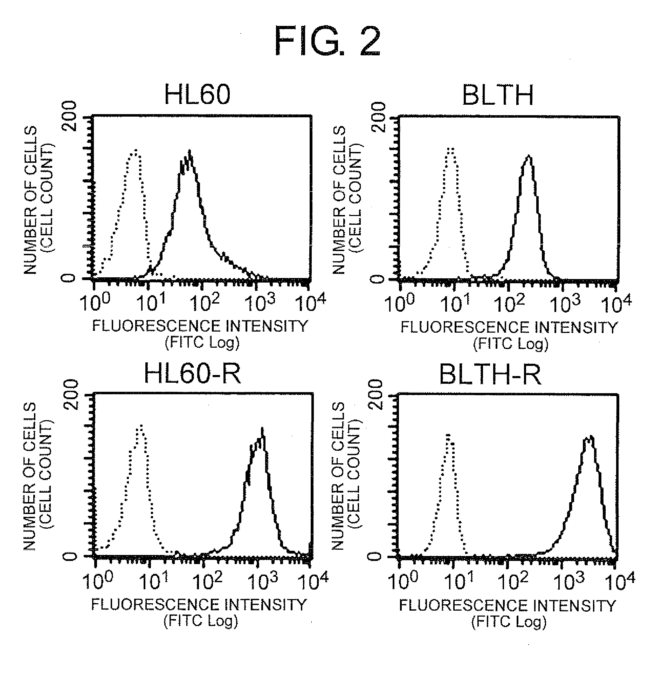

Fig. 2 is composed of a set of graphs depicting the results of flow cytometry assays measuring the over-expression of the HLA class I molecule in chemotherapeutic agent-resistant cell lines. The dotted lines and the solid line indicate the measured results obtained using a control antibody and the anti-C3B3 IgG antibody (full length C3B3 antibody), respectively.

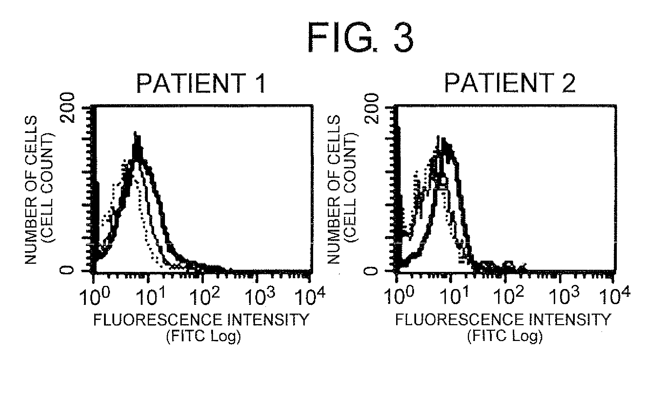

Fig. 3 is composed of a set of graphs depicting the results of flow cytometry assays measuring MDR1 expression in tumor cells derived from acute myeloid leukemia (AML) patients, both at the first medical examination and at the time of recurrence. The dotted lines and the solid lines indicate the measured results obtained using a control antibody and the anti-MDR1 antibody at the first medical examination, respectively, and the bold lines indicate the measured results obtained using anti-MDR1 antibody at the time of recurrence.

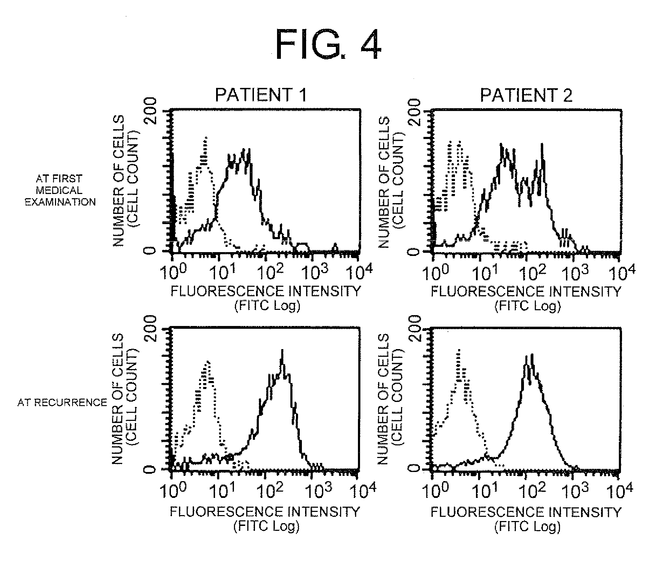

Fig. 4 is composed of a set of graphs depicting the results of flow cytometry assays measuring expression of the HLA class I molecule in tumor cells derived from acute myeloid leukemia (AML) patients, both at the first medical examination and at the time of recurrence. The dotted lines and the solid lines indicate the measured results obtained using a control antibody and the anti-C3B3 IgG antibody (full length C3B3 antibody), respectively.

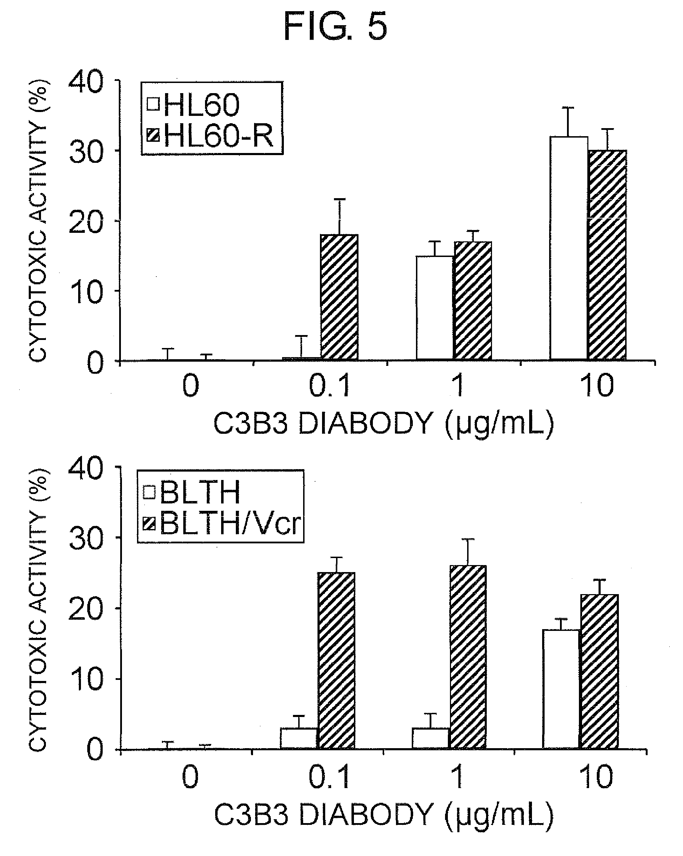

Fig. 5 is composed of a set of graphs demonstrating the cytotoxic activity of a C3B3 diabody in chemotherapeutic agent-resistant cell lines.

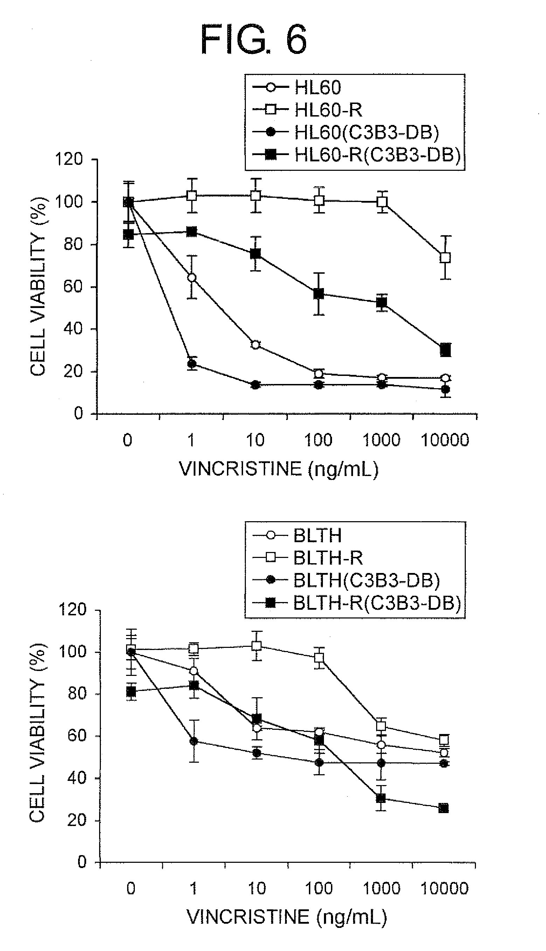

Fig. 6 is composed of a set of graphs depicting the enhancement action of a C3B3 diabody on the cytotoxic activity of vincristine in chemotherapeutic agent-resistant cell lines.

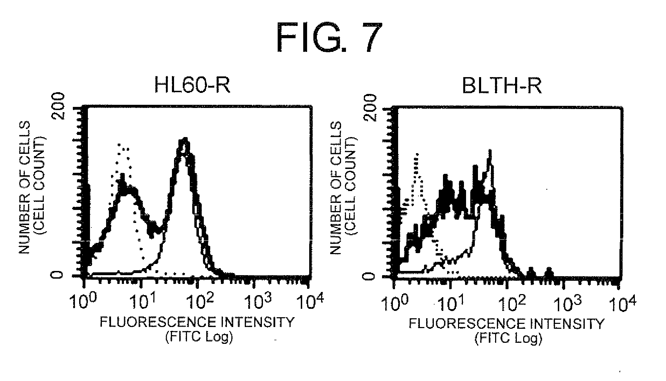

Fig. 7 is composed of a set of graphs depicting MDR1 expression level on the cell surface after C3B3 diabody treatment in chemotherapeutic agent-resistant cell lines. The dotted lines, the solid lines, bold lines indicate the measured results obtained using a control antibody, the anti-MDR1 antibody, and the anti-MDR1 antibody after C3B3 diabody treatment, respectively.

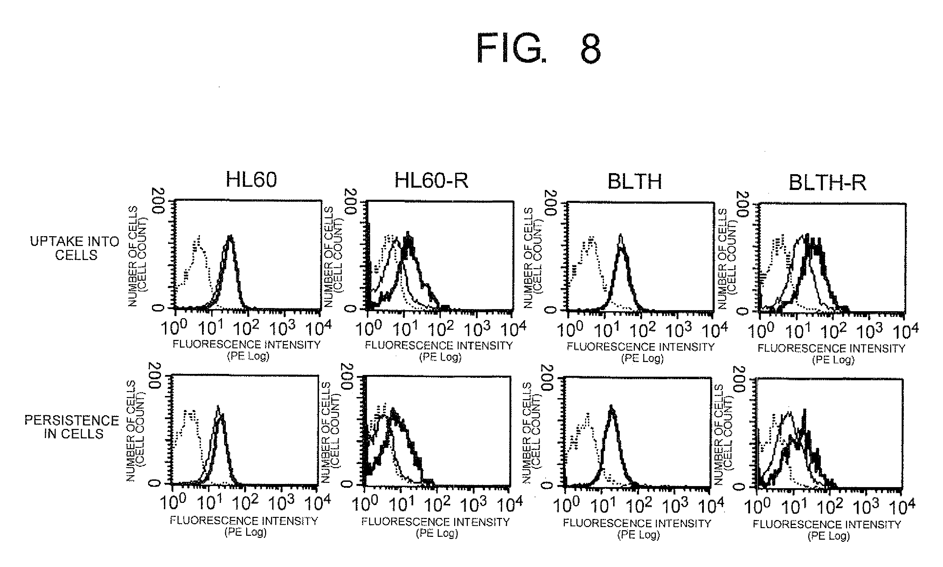

Fig. 8 is composed of set of graphs demonstrating that C3B3 diabody treatment leads to a recovery of pharmaceutical agent retaining activity in chemotherapeutic agent-resistant cell lines. The dotted lines, the solid lines, and the bold lines indicate the results obtained by adding a control, daunorubicin alone, and daunorubicin after anti-C3B3 diabody treatment.

### Examples

Herein below, the present invention will be specifically described with reference to Examples, but should not to be construed as being limited thereto.

### [Example 1] Establishment of chemotherapeutic agent (vincristine)-resistant hematological tumor cell lines

Acute myeloid leukemia cell line HL60 (American Type Culture Collection, Manassas, VA, USA) and Burkitt's lymphoma cell line BLTH (kindly gifted from Prof. Hirose at The University of Tokushima, Br. J. Cancer (1987) 56: 413-417) were cultured in the presence of vincristine to obtain vincristine-resistant cell lines HL60-R and BLTH-R.

### [Example 2] Confirmation of mdr1 mRNA and the amount of HLA class I expression in tumor cell lines

First, mdr1 mRNA expression in HL60, HL60-R, BLTH, and BLTH-R was confirmed by RT-PCR, and the expression of the HLA class I protein and the MDR1 protein on the cell surface were confirmed by flow cytometry.

mar1 mRNA expression in these cells were examined by RT-PCR using specific primers (5'-CCC ATC ATT GCAATA GCA GG (SEQ ID NO: 13) and 3'-GTT CAAACT TCT GCT CCT GA (SEQ ID NO: 14)). As a control, expression of β2-microglobulin mRNA expression was examined using specific primers (5'-ACC CCC ACT GAAAAA GAT GA (SEQ ID NO: 15) and 3'-ATC TTC AAA CCT CCA TGA TG (SEQ ID NO: 16)).

MDR1 expression on the cell surface was examined by flow cytometry using anti-MDR1 antibody (UIC-2, Chemicon, Temecula, CA, USA) and FITC-labeled goat anti-mouse IgG antibody (Biosource, Camarillo, CA, USA). Mouse IgG (BD Biosciences, San Jose, CA, USA) was used for the control antibody.

As a result, the mdr1 mRNA expression was not observed in the parent cell lines, HL60 and BLTH; however, it was observed in vincristine-resistant cell lines, HL60-R and BLTH-R (Fig. 1).

HLA class I expression intensities on the surface of these cells were also compared. Expression of HLA class I was determined by flow cytometry using Alexa 488 (Molecular Probes, Eugene, OR, USA)-labeled C3B3 antibody. The results showed that the expression of HLA class I on the cell surface is enhanced in both vincristine-resistant cell lines (Fig. 2).

Analyses by flow cytometry were performed similarly on tumor cells obtained at the first medical examination and at recurrence from acute myeloid leukemia patients to confirm the amount of MDR1 and HLA class I expression on the tumor cell surface. As a result, when comparing to cells obtained at the first medical examination, cells obtained at recurrence showed induction of MDR1 expression (Fig. 3) and enhancement of HLA class I expression (Fig. 4).

### [Example 3] Cytotoxic activity of C3B3-DB in tumor cell lines

First, these tumor cells were used to examine their susceptibility to C3B3-DB. These tumor cells were cultured in the presence of various concentrations of C3B3-DB and then antitumor activity (cytotoxic activity) of C3B3-DB was measured by cell growth tests using WST-8 (Kishida Chemicals, Osaka).

Culturing these cells for 24 hours in the presence of C3B3-DB and comparing the survival rate of the cells demonstrated that cell injury was induced at lower concentrations of C3B3-DB in the vincristine-resistant cell lines, HL60-R and BLTH-R (Fig. 5).

In addition, whether or not drug resistance in chemotherapeutic agent-resistant tumor cells can be overcome (improved) was examined by culturing these cells in the presence or absence of C3B3-DB (0.1 µg/mL) for six hours and then adding vincristine. In the parent cell lines, HL60 and BLTH, concentration-dependent cell injury by vincristine was induced regardless of whether or not pre-treatment with C3B3-DB was carried out, and effects of cytotoxic activity enhancement of vincristine due to pre-treatment with C3B3-DB was not observed. On the other hand, in HL60-R and BLTH-R, cytotoxic activity of vincristine was found to be enhanced by pre-treatment with C3B3-DB (Fig. 6).

### [Example 4]

To elucidate the mechanism of overcoming drug (chemotherapeutic agent)-resistance using C3B3-DB, MDR1 expression on the cell surface was examined after treating these cells for six hours with C3B3-DB (0.1 µg/mL). MDR1 expression was found to decrease in some cells treated with C3B3-DB (Fig. 7). Furthermore, treatment of these cells with C3B3-DB was followed by culturing these cells in the presence of daunorubicin (0.1 µg/mL, Meiji Seika, Tokyo) and the amount of daunorubicin uptake into cells were compared.

Specifically, uptake of the pharmaceutical agent into the cells (accumulation phase) was evaluated by culturing the cells for two hours in the presence or absence of C3B3-DB, then adding daunorubicin and culturing the cells for 30 minutes, washing the cells, and then measuring the PE intensity by flow cytometry. The cells were cultured for another 30 minutes to evaluate persistence of the pharmaceutical agent in the cells (efflux phase) in a same manner using flow cytometry.

As a result, in the parent cell lines, a certain amount of daunorubicin was taken up in both the accumulation phase and efflux phase, and pre-treatment effects with C3B3-DB were not observed. On the other hand, in the vincristine-resistant cell lines, the amount of daunorubicin uptake was significantly decreased in both phases but it was found that the pre-treatment with C3B3-DB increased the amount of uptake (Fig. 8).

The above confirmed that in drug-resistant cell lines and tumor cells derived from patients with recurrent acute myeloid leukemia, MDR1 expression on the cell surface is induced, while expression of HLA class I is enhanced simultaneously. Transporter function of MDR1 may be involved in the strong HLA class I expression mechanism.

C3B3-DB specifically induced cell injury caused by the antibody alone against drug-resistant tumor cells highly expressing HLA class I. When combined with a chemotherapeutic agent, C3B3-DB enhanced the cytotoxic activity of the pharmaceutical agent. Regarding its mechanism, C3B3-DB was found to enhance the amount of the chemotherapeutic agent taken up into cells by decreasing MDR1 expression in these cells.

According to the above-mentioned results, drug resistance of MDR1-expressing tumor cells can be overcome by the combined use of C3B3-DB and a chemotherapeutic agent.

### Industrial Applicability

The present invention provides therapeutic agents for chemotherapeutic agent-resistant cancer, such agents including as an active ingredient, an HLA class I-recognizing antibody. The present invention also provides methods for treating chemotherapeutic agent-resistant cancer, such methods including the step of administering an HLA class I-recognizing antibody to a subject.

In the context of chemotherapy for malignant tumors, an important objective is to develop therapeutic strategies to overcome drug resistance in MDR1-expressing tumors which cause reduction of chemotherapeutic effects. The therapeutic agents of the present invention provide immunological therapeutic methods that target HLA class I molecules, even for the drug-resistant MDR1-expressing tumors.

## Claims

1. A therapeutic agent for chemotherapeutic agent-resistant cancer, comprising an HLA class I-recognizing antibody as an active ingredient.

2. The therapeutic agent for chemotherapeutic agent-resistant cancer of claim 1, wherein the antibody is a low-molecular-weight antibody.

3. The therapeutic agent for chemotherapeutic agent-resistant cancer of claim 1 or 2, in combination with a chemotherapeutic agent.

4. The therapeutic agent for chemotherapeutic agent-resistant cancer of any one of claims 1 to 3, wherein the chemotherapeutic agent-resistant cancer is a hematological tumor.

5. A potentiator of a chemotherapeutic agent comprising an HLA class I-recognizing antibody as an active ingredient.

6. The potentiator of a chemotherapeutic agent of claim 5, wherein the antibody is a low-molecular-weight antibody.

7. The potentiator of a chemotherapeutic agent of claim 5 or 6, in combination with a chemotherapeutic agent.

8. The potentiator of a chemotherapeutic agent of any one of claims 5 to 7, wherein the cancer treated by a chemotherapeutic agent is a chemotherapeutic agent-resistant cancer.

9. The potentiator of a chemotherapeutic agent of claim 8, wherein the chemotherapeutic agent-resistant cancer is a hematological tumor.

10. A pharmaceutical composition comprising an HLA class I-recognizing antibody as an active ingredient, in combination with a chemotherapeutic agent.

11. A pharmaceutical composition for cancer therapy comprising an HLA class I-recognizing antibody as an active ingredient, in combination with a chemotherapeutic agent.

12. The pharmaceutical composition of claim 10 or 11, wherein the antibody is a low-molecular-weight antibody.

13. A pharmaceutical composition for cancer therapy comprising a chemotherapeutic agent and an HLA class I-recognizing antibody as active ingredients.

14. The pharmaceutical composition for cancer therapy of claim 13, wherein the antibody is a low-molecular-weight antibody.

15. The pharmaceutical composition of any one of claims 10 to 14, wherein the cancer to be treated with a chemotherapeutic agent is a chemotherapeutic agent-resistant cancer.

16. The pharmaceutical composition of claim 15, wherein the chemotherapeutic agent-resistant cancer is a hematological tumor.

17. A method for treating chemotherapeutic agent-resistant cancer, wherein the method comprises the step of administering an HLA class I-recognizing antibody to a subject.

18. The method of claim 17, wherein the antibody is a low-molecular-weight antibody.

19. The method of claim 17 or 18, wherein a chemotherapeutic agent is used in combination.

20. The method of any one of claims 17 to 19, wherein the chemotherapeutic agent-resistant cancer is a hematological tumor.

21. A method for strengthening the effect of a chemotherapeutic agent, wherein the method comprises the step of administering an HLA class I-recognizing antibody to a subject.

22. The method of claim 21, wherein the antibody is a low-molecular-weight antibody.

23. The method of claim 21 or 22, wherein the chemotherapeutic agent is used in combination.

24. The method of any one of claims 21 to 23, wherein a cancer to be treated with a chemotherapeutic agent is a chemotherapeutic agent-resistant cancer.

25. The method claim 24, wherein the chemotherapeutic agent-resistant cancer is a hematological tumor.
